# EUROPEAN PATENT SPECIFICATION

(11) **EP 0 918 543 B1**
(45) Date of publication and mention of the grant of the patent: **21.04.2004**
(21) Application number: 97935194.7
(22) Date of filing: 30.07.1997
(51) Int. Cl.: A61K 47/32

(54) **POLYMER COMPOSITION FOR ORAL ADMINISTRATION OF PEPTIDES AND PROTEINS**
POLYMERZUSAMMENSETZUNG ZUR ORALEN ADMINISTRATION VON PEPTIDEN UND PROTEINEN
COMPOSITION POLYMERE POUR L'ADMINISTRATION DE PEPTIDES ET PROTEINES A VOIE ORALE

(30) Priority: 02.08.1996 US 691617
(43) Date of publication of application: 02.06.1999
(73) Proprietor: Orex Pharmaceutical Development Corp., St. Louis, MO 63141 (US)
(72) Inventor: PLATE, Nikolai A., Moscow, 117334 (RU); VALUEV, Lev I., Moscow, 127238 (RU); VALUEVA, Tatyana A., Moscow, 127238 (RU); STAROSELTSEVA, Ludmila K., Moscow, 109382 (RU); AMETOV, Alexander S., Moscow, 129515 (RU); KNYAZHEV, Vladimir A., Moscow, 107113 (RU); HENIS, Jay, M., S., St. Louis, MO 63141 (US)
(74) Representative: von Kreisler, Alek, Dipl.-Chem.
(86) International application number: PCT/US1997/013352
(87) International publication number: WO 1998/005362

(56) References cited:
- EP-A- 0 098 110
- WO-A-95/15352
- DE-A- 19 510 551
- RU-C- 2 076 733
- US-A- 5 049 545
- US-A- 5 359 030
- VALUEV L I ET AL: "IMMOBILIZATION OF OVOMUCOID ON DEXTRANS" POLYMER SCIENCE, vol. 34, no. 11, 1 January 1992, pages 972-974, XP000336191
- VALUEV L I ET AL: "INTERACTION OF POLYMERIC DERIVATIVES OF TRYPSIN WITH ITS PROTEIN INHIBITOR" POLYMER SCIENCE: SERIE A, vol. 37, no. 9, 1 September 1995, pages 910-914, XP000540705
- KOPECEK J. ET AL: "Polymers for colon-specific drug delivery" J. CONTROL. RELEASE, 1992, 19/1-3 (121-130), NETHERLANDS, XP002057830

## Description

### FIELD OF THE INVENTION

The invention relates to a therapeutic-containing composition adapted for the oral administration of a biologically active material other than insulin which therapeutic proteins and peptides ordinarily are not easily delivered orally. The formulation may consist of a conventional chemical compound, a protein, a peptide, or a peptide bio-mimetic incorporated within a hydrogel whose polymer structure has been chemically modified to include a functionality which protects the therapeutic from degradation, and a functionality which interacts with the stomach or intestinal mucosa to favorably increase the probability of the therapeutic diffusing into the circulatory system. More specifically the invention deals with a therapeutic containing composition adapted for the oral administration of a biologically active material other than insulin, this composition comprising a water swellable polymer chemically modified with an enzyme inhibitor and a protein, peptide, or peptide bio-mimetic, such as growth hormone and erythropoietin, as well as other peptides whose molecular weight is in the range of 30,000 or other proteins having a molecular of up to one million, for oral delivery in treating respectively various forms and degrees of various growth hormone related diseases and various blood related diseases. Other uses specifically include the treatment of animal diseases and other biological applications involving growth hormones and related analogies and metabolites for improved dairy and/or meat production.

### BACKGROUND OF THE INVENTION

Since the advent of biotechnology many protein therapeutics have been identified as having significant therapeutic benefits for many potentially serious diseases. Such diseases include cancer, heart disease, diseases of the central nervous system, diseases of the immune system, diseases of the blood and circulatory system, and many hormonally based diseases. Unfortunately, many of these medical disorders are chronic and require continuous administration of the required therapeutic.

However, the broad utilization of protein therapeutics, and many other hard to deliver therapeutics depends on the ability to easily administer them to patients in a controllable and acceptable manner. Clearly oral administration would be the most desirable method for administering such materials to patients who must take them for extended periods of time. Unfortunately, the ability to administer proteins in this fashion has never been achieved because typically proteins and peptides are easily metabolized, partially degraded and/or can formationally modified in the stomach and intestine when introduced orally to patients. In addition to problems related to degradation, their molecular weights are typically too high to allow for significant transport across the gut wall into the circulatory system. This is true even if they can be protected from extensive degradation in the stomach and/or intestine and can approach the gut wall in essentially biochemically unaltered form. As a result protein therapeutics can only be given by injection (typically intravenous (IV) or subcutaneous), and even low molecular weight peptides and peptide mimetics cannot be practically administered orally. This has severely restricted their broad utilization. for many diseases.

Indeed, an approach which would permit the direct oral delivery of proteins and peptides has been sought for many years, as it has been generally recognized that such an approach, if developed, would have important advantages and thus greatly expand the potential uses for protein and peptide therapeutics. Nevertheless, and in spite of extensive efforts to develop insulin and other proteins and peptides, preparations resistant to the action of various digestive proteinases and capable of delivery into the blood stream through the intestinal mucosa, no such approach has been found and used for the general administration of either insulin or other protein or peptide therapeutics up to now.

Indeed, the disadvantages of administration by injection of most protein therapeutics, has been one of the principle elements limiting the use of proteins, as therapeutics for chronic diseases. The same problems and limitations apply to the use of proteins in animals where multiple and often daily repetitive doses would be required to achieve the necessary biological effect, and to non-protein therapeutics which are not orally bioavailable.

The compositions and method of this invention relates to any number of potentially efficacious proteins and peptides as well as other therapeutics. The oral delivery of growth hormones are used to illustrate the general applicability of the methodology and the formulations described. We show animal studies, formulations, and a methodology for making the formulations which permits the creation of preparations resistant to the action of digestive proteinase. The use of these formulations permits the contained therapeutic protein to penetrate the intestinal mucosa and enter the circulatory system where it can manifest its therapeutic effect. We show further that therapeutic efficacy can be achieved with dosage levels of insulin which are comparable to the dosage levels commonly used in conventional therapeutic injections.

US Patent No. 5049545 discloses injectable insulin-containing compositions which are comprised of insulin immobilized on a polymer (including a polymer hydrogel), such as starch, dextran, polyoxyethylene, polyvinylpyrrolidone, and these formulations have included inhibitors of proteolytic enzymes. However, the insulin-containing polymer compositions synthesized in this work do not show significant stability to the attack of the digestive enzymes and hence are not useful for oral delivery.

Saffran M., Kumar G.S., et al. Biochem. Soc. Trans. 1990, v.18, N. 5, p.752; Damge, C., J Controlled Release, 1990, V. 13. p. 233; and Greenley R.Z., Broun T. M. et al. Polymer Prepr., 1990, v. 31, N 2. p.182. have reported various formulations containing a polymer and insulin, but the resulting compositions are not sufficiently effective to deliver insulin to the blood stream when administered orally.

German Patent Application Offenbegunsschrift DE 195 10 551 A1, published 28 September 1995, discloses an oral delivery of insulin which comprises a hydrophilic polymer modified with ovomucoid isolated from duck or turkey egg white (which is an inhibitor of proteolytic enzymes), insulin and water. There is no suggestion that any other proteins may be delivered in such manner or that any other enzyme inhibitor immobilized to a gel matrix could or would facilitate such delivery. In fact, one could not predict that other biologically active proteins or peptides may be administered employing such a formulation because proteins and peptides are complex molecules varying from one another in chemical composition and in physical properties.

It is well recognized that there are many barriers and difficulties associated with the oral delivery of proteins or peptides. These include chemical and enzymatic degradation which takes place in different parts of the digestive tract, the barriers to transport represented by the intestinal mucosa and specific enzymes contained therein, and the even greater cellular barriers and tight junctions of the intestinal lining (Wei Wang J. of Drug Targeting, (1996) Vol. 4, No. 4 pp. 195-232). In spite of such barriers there is ample evidence that some nutritional and other proteins can pass through these barriers to a limited degree. Such differences in protein behavior, barriers to transport, and related problems have made the practical delivery of protein or protein like biologically active materials by the oral route unattainable to date (Elke Lipka, John Crison, Gordon Amidon, J. Controlled Release (1996) 39, 121-129). Fucthermore, it is known that there exist specific amino acid, and di- and tri-peptide transport mechanisms as well as receptors and transport mechanisms for specific proteins and peptides in different parts of the intestinal system (US Patent 5438040 (Ekwuribe) and P. Smith "Exploitation of the Intestinal Oligopeptide Transporter to Enhance Drug Absorption," Vol. 3 No. 2, 1996). Dressman and Berardi² J. Pharm. Sci. 82(9), 1993, pg. 857, point out that there are many GI parameters such as pH, brush border and microcolonic activity which play key roles in the amount and state of therapeutic molecules to be delivered orally. They show that while many specific mechanisms and approaches have been proposed, none have been widely applicable and each is tied to a specific approach.

There are a wide range of degrading enzymes such as pepsidases and proteases located in different regions of the intestinal track and active to a greater or lesser degree under differing conditions against different kinds of proteins. It can be expected that all of these conditions must be separately considered and manipulated when attempting to successfully deliver each hard to deliver and unstable protein drug.

The multiplicity of properties, individual molecular parameters, and unique potential transport pathways, which can impact the survival and delivery of a protein have resulted in many different strategies for delivery of such molecules. It is therefore understandable that the literature in this area does not suggest a general mechanism which can more or less uniformly effect the oral delivery of a wide spectrum of proteins, with widely varying molecular weights, without chemically altering the molecule of a drug. While approaches have been designed which depend on modifying protein drugs with specific groups and functionalities which can take advantage of such specific and selective transport mechanisms, these efforts have serious problems, and have not been found to be either generally or practically successful. What is clear however, is that even proteins which differ from each other by only a few amino acid sequences out of hundreds or even thousands of such sequences can be very different in biological and/or stability properties. Hence, any system which can be shown to affect similar delivery results across a range of such drugs must be considered as unique, and the broadly consistent in-vitro and in-vivo results reported here is hardly to be expected.

### SUMMARY OF THE INVENTION

It is a general object of the invention to provide a composition for oral administration of therapeutic and bioactive materials other than insulin whose oral availability is sufficiently limited under normal circumstances that they cannot be effectively utilized with this route of administration. More specifically, this invention deals with a composition comprising a hydrophilic polymer chemically modified with an enzyme inhibitor and a therapeutical material other than insulin, often a protein or peptide, said composition being adequate for oral administration.

A further object of the invention is to provide a therapeutic-containing composition for oral administration which contains a water swellable polymer modified with an enzyme inhibitor and a therapeutic material, protein or peptide other than insulin, said composition being in a tablet or capsule form, typically such that it may safely pass through the gastric environment and release the therapeutic ingredient in the small intestines.

A still further object of the invention is to increase the resistance of protein or peptide therapeutics to the effects of various digestive enzymes so as to enable such therapeutics to be more effectively administered orally.

A further object of the invention is to increase the interaction of the therapeutic-containing polymer hydrogels with selected transport barriers, and interactive sites of the intestine and/or bowels to increase the efficacy of the therapeutic substances.

A further object of the invention is to physically incorporate and entrap within the chemically modified hydrogels a protein or a peptide or other hard to deliver therapeutics without reacting, binding or substantially modifying such therapeutics, to properly protect them from premature decomposition, degradation, modification or destruction prior to its delivery to the desired location in the body such as to the intestinal barrier.

A further object of the invention is to provide a composition suitable to deliver a therapeutically useful amount of a protein or a peptide other than insulin to the target location in humans or animals to achieve useful therapeutic effects at such dosing levels that may produce desirable results similar to those when such therapeutic materials are injected into the body and/or at levels significantly lower than would be required when the therapeutics are given orally in the absence of this invention.

These and other objects are achieved by compositions more specifically described below.

### BRIEF DESCRIPTION OF THE DRAWINGS

Figure 1 is a drawing of the setup used to study the protein/peptide diffusion from the particles of polymeric hydrogels;
Figure 2, 3 and 4 are graphs of the data reported in Tables 9-15.

### DETAILED DISCLOSURE OF THE INVENTION

The invention is directed to
(1) A therapeutic-containing composition adapted for the oral administration of a biologically active material other than insulin comprising
   (a) a water insoluble, but water swellable, hydrophilic polymer which is chemically modified to contain at least one proteolytic enzyme inhibitor and a chemical functionality which has an interactive affinity for target receptors located on the transport barrier walls of the digestive track of the intended animal specie, which chemical functionality is a lectin binding agent or a glycoside;
   (b) one or more therapeutic materials selected from a growth hormone, an interleukin, a blood cell growth stimulating factor, erithropoitin (EPO), parathyroid hormone (PTH), selenoprotein P, cystatin B megakaryocyte simulatory factor (MGDF), granulocyte macrophage colony stimulating factor, tumor invasion inhibiting factors, transacting regulatory proteins (TAT's) of HIV and other retroviruses, and transactivating regulator protein BPC 157, fat reducing hormones, calcitonin, glucagon; and
   (c) water,
and (2) A method of preparing a composition of item 1 comprising the steps of
   (a) functionalizing a proteolytic enzymes inhibitor by reacting said inhibitor with a compound having a polymerizable vinyl group and a functional group which is reactive with said inhibitor,
   (b) polymerizing a mixture comprising 75 to 99 weight percent of one or more polymerizable monomers, 0.1 to 20 weight percent of a crosslinking agent and 0.01 to 5 weight percent of a functionalized proteolytic enzyme inhibitor obtained in step (a) to yield a hydrophilic polymer, and
   (c) immersing the hydrophilic polymer in an aqueous solution of a therapeutic material selected from a growth hormone, an interleukin, a blood cell growth stimulating factor, erithropoitin (EPO), parathyroid hormone (PTH), selenoprotein P, cystatin B, megakaryocyte simulatory factor (MGDF), granulocyte macrophage colony stimulating factor, tumor invasion inhibiting factors, transacting regulatory proteins (TAT's) of HIV and other retroviruses, and transactivating regular protein BPC 157, fat reducing hormones, calcitonin and glucagons.

The compositions of this invention, when administered orally exhibit at least 25 % of the biological efficacy of the delivered therapeutic compared to the efficacy when the therapeutic is administered by either intravenous or subcutaneous injection.

The polymers useful in this invention are desirably water interactive and/or hydrophilic in nature and are of a molecular weight or structures, or have been modified such that they absorb significant quantities of water and may form hydrogels when placed in contact with water or aqueous media for a period of time sufficient to reach equilibrium with water, but which do not fully dissolve at equilibrium in water. For the purpose of specificity, we shall define a hydrogel of the invention as a polymer matrix which, when placed in contact with an excess of water, absorbs at least two times its weight of water at equilibrium when exposed to water at room temperature.

Such polymers should preferably be stable themselves and form stable hydrogels in a range of pH conditions ranging from pH 1 to pH 10 and most preferably be stable under pH conditions ranging from at least 3 to 9 without additional protective coatings. However, the particularly desirable stability properties must be tailored to the site of required delivery, and there may be specific times at which higher or lower stabilities to a particular pH and chemical or biological environment will be most desirable. Thus, there may be situations where susceptibility to degradation at a selected pH or in the presence of a specific hydrolytic enzyme or other degrading agent may be useful in achieving a particular delivery profile. Therefore, the formulation of the invention must be stable under conditions varying from those of the stomach to those of the site of delivery for a period of at least 2 times the normal transport time to the site of delivery in the host animal for which the therapeutic or bioactive material is intended.

The polymers of the invention may preferably include polymers from the group of homo- and copolymers based on various combinations of the following vinyl monomers: acrylic and methacrylic acids, acrylamide, methacrylamide, hydroxyethylacrylate or methacrylate, vinylpyrrolidones, as well as polyvinylalcohol and its co- and terpolymers, polyvinylacetate, its co- and terpolymers with the above listed monomers and 2-acrylamido-2-methylpropanesulfonic acid (AMPS®) and sulfonated styrenes. Very useful are copolymers of the above listed monomers with copolymerizable functional monomers such as acryl or methacryl amide acrylate or methacrylate esters where the ester groups are derived from straight or branched chain alkyl, aryl having up to four aromatic rings which may contain alkyl substituents of 1 to 6 carbons; steroidal, sulfates, phosphates or cationic monomers such as N,N-dimethylaminoalkyl(meth)acrylamide, dimethylaminoalkyl(meth)acrylate, (meth)acryloxyalkyltrimethylammonium chloride, (meth)acryloxyalkyldimethylbenzyl ammonium chloride. Further useful polymers are those classified as dextrans, dextrins, and from the class of materials classified as natural gums and resins, or from the class of natural polymers such as processed collagen, chitin, chitosan, pullalan, zooglan, alginates and modified alginates such as "Kelcoloid" (a polypropylene glycol modified alginate) gellan gums such as "Kelocogel", Xanathan gums such as "Kestrol", estastin, alpha hydroxy butyrate and its copolymers, hyaluronic acid and its derivatives, polylactic and glycolic acids and their derivatives and the like including chemically and physically modified versions of these polymers, gums and resins, and blends of these materials with each other or other polymers so long as the alterations, modifications or blending do not cause the final properties to fall below the minimum set for water absorption, hydrogel formation and the chemical stability required for useful application. Also polymers such as nylon, acrylan or other normally hydrophobic synthetic polymers may be sufficiently modified by reaction to become water swellable and/or form stable gels in aqueous media. In such modified form such polymers also may be acceptable for the oral delivery of some proteins if the formulations for which they are the matrix follow the other teachings of the invention.

A very useful class of polymers applicable in this invention are the carboxylic monomers are the olefinically-unsaturated carboxylic acids containing at least one activated carbon-to-carbon olefinic double bond, and at least one carboxyl group; that is, an acid or function readily converted to an acid containing an olefinic double bond which readily functions in polymerization because of its presence in the monomer molecule, either in the alpha-beta position with respect to a carboxyl group, -C=C-COOH; or as part of a terminal methylene grouping, CH₂=C <. Olefinically-unsaturated acids of this class include such materials as the acrylic acids typified by the acrylic acid itself, alpha-cyano acrylic acid, beta methylacrylic acid (crotonic acid), alpha-phenyl acrylic acid, beta-acryloxy propionic acid, cinnamic acid, p-chloro cinnamic acid, 1-carboxy-4-phenyl butadiene-1,3, itaconic acid, citraconic acid, mesaconic acid, glutaconic acid, aconitic acid, maleic acid, fumaric acid, and tricarboxy ethylene. As used herein, the term "carboxylic acid" includes the polycarboxylic acids and those acid anhydrides, such as maleic anhydride, wherein the anhydride group is formed by the elimination of one molecule of water from two carboxyl groups located on the same carboxylic acid molecule. Maleic anhydride and other acid anhydrides useful herein have the general structure 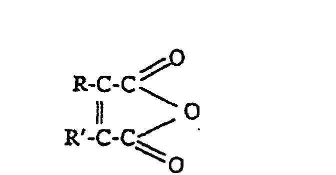 wherein R and R' are selected from the group consisting of hydrogen, halogen and cyanogen (-C≡N) groups and alkyl, aryl, alkaryl, aralkyl, and cycloalkyl groups such as methyl, ethyl, propyl, octyl, decyl, phenyl, tolyl, xylyl, benzyl, and cyclohexyl.

The preferred carboxylic monomers are the monoolefinic acrylic acids having the general structure 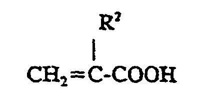 wherein R² is a substituent selected from hydrogen, halogen, and the cyanogen (-C ≡ N) groups, monovalent alkyl radicals, monovalent aryl radicals, monovalent aralkyl radicals, monovalent alkaryl radicals and monovalent cycloaliphatic radicals. Of this class, acrylic and methacrylic acid are most preferred. Other useful carboxylic monomers are maleic acid and its anhydride.

The polymers include both homopolymers of carboxylic acids or anhydrides thereof, or the defined carboxylic acids copolymerized with one or more other vinylidene monomers containing at least one terminal > CH₂ group. The other vinylidene monomers are present in an amount of less than 30 weight percent based upon the weight of the carboxylic acid or anhydride plus the vinylidene monomer(s). Such monomers include, for example, acrylate ester monomers including those acrylic acid ester monomers such as derivatives of an acrylic acid represented by the formula 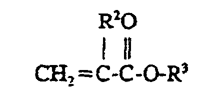 wherein R³ is an alkyl group having from 1 to 30 carbon atoms, preferably 1 to 20 carbon atoms and R² is hydrogen, methyl or ethyl, present in the copolymer in amount, for example, from about 1 to 40 weight percent or more. Representative acrylates include methyl acrylate, ethyl acrylate, propyl acrylate, isopropyl acrylate, butyl acrylate, isobutyl acrylate, methyl methacrylate, methyl ethacrylate, ethyl methacrylate, octyl acrylate, heptyl acrylate, octyl methacrylate, isopropyl methacrylate, 2-ethylhexyl methacrylate, nonyl acrylate, hexyl acrylate, n-hexyl methacrylate, and the like. Higher alkyl acrylic esters are decyl acrylate, isodecyl methacrylate, lauryl acrylate, stearyl acrylate, behenyl acrylate and melissyl acrylate and methacrylate versions thereof. Mixtures of two or three or more long chain acrylic esters may be successfully polymerized with one of the carboxylic monomers. Other comonomers include olefins, including alpha olefins, vinyl ethers, vinyl esters, and mixtures thereof.

Other vinylidene monomers may also be used, including the acrylic nitriles. The useful α,β-olefinically unsaturated nitriles are preferably the monoolefinically unsaturated nitriles having from 3 to 10 carbon atoms such as acrylonitrile, methacrylonitrile. Most preferred are acrylonitrile and methacrylonitrile. The amounts used may vary, but for some polymers are, for example from about 1 to 30 weight percent of the total monomers copolymerized. Acrylic amides containing from 3 to 35 carbon atoms including monoolefinically unsaturated amides also may be used. Representative amides include acrylamide, methacrylamide, N-t-butyl acrylamide, N-cyclohexyl acrylamide, higher alkyl amides, where the alkyl group on the nitrogen contains from 8 to 32 carbon atoms, acrylic amides including N-alkylol amides of alpha, beta-olefinically unsaturated carboxylic acids including those having from 4 to 10 carbon atoms such as N-methylol acrylamide, N-propanol acrylamide, N-methylol methacrylamide, N-methylol maleimide, N-methylol maleamic acid esters, N-methylol-p-vinyl benzamide, and the like. Still further useful materials are alpha-olefins containing from 2 to 18 carbon atoms, more preferably from 2 to 8 carbon atoms; dienes containing from 4 to 10 carbon atoms; vinyl esters and allyl esters such as vinyl acetate; vinyl aromatics such as styrene, methyl styrene and chlorostyrene; vinyl and allyl ethers and ketones such as vinyl methyl ether and methyl vinyl ketone; chloroacrylates; cyanoalkyl acrylates such as α-cyanomethyl acrylate, and the α-, β-, and γ-cyanopropyl acrylates; alkoxyacrylates such as methoxy ethyl acrylate; haloacrylates as chloroethyl acrylate; vinyl halides and vinyl chloride, and vinylidene chloride divinyls, diacrylates and other polyfunctional monomers such as divinyl ether; diethylene glycol diacrylate, ethylene glycol dimethacrylate, methylene-bis-acrylamide, and allylpentaerythritol; and bis (β-haloalkyl) alkenyl phosphonates such as bis(β-chloroethyl) vinyl phosphonate as are known to those skilled in the art. Copolymers wherein the carboxy containing monomer is a minor constituent, and the other vinylidene monomers present as major components are readily prepared in accordance with the process of this invention.

Most preferably the hydrogels of the invention should be composed of synthetic copolymers from the group of acrylic and methacrylic acids, acrylamide, methacrylamide, hydroxyethylacrylate (HEA) or methacrylate (HEMA), and vinylpyrrolidones which are water interactive and swellable. Specific illustrative examples of useful polymers, especially for the delivery of insulin or growth hormones, are the following types of polymers:
(meth)acrylamide and 0.1 to 99 wt. % (meth)acrylic acid;
(meth)acrylamides and 0.1 - 75 wt % (meth)acryloxyethyl trimethyammonium chloride;
(meth)acrylamide and 0.1 - 75 wt % (meth)acrylamide;
acrylic acid and 0.1 - 75 wt % alkyl(meth)acrylates;
(meth)acrylamide and 0.1 - 75 wt % AMPS® (trademark of Lubrizol Corp.);
(meth)acrylamide and 0 to 30 wt % alkyl(meth)acrylamides and 0.1 - 75 wt % AMFS®;
(meth)acrylamide and 0.1 - 99 wt. % HEMA;
(meth)acrylamide and 0.1 to 75 wt % HEMA and 0.1 to 99 % (meth)acrylic acid;
(meth)acrylic acid and 0.1 - 99 wt % HEMA;
50 mole % vinyl ether and 50 mole % maleic anhydride;
(meth)acrylamide and 0.1 to 75 wt % (meth)acryloxyalky dimethyl benzylammonium chloride;
(meth)acrylamide and 0.1 to 99 wt % vinyl pyrrolidone;
(meth)acrylamide and 50 wt % vinyl pyrrolidone and 0.1 - 99.9 wt % (meth)acrylic acid;
(meth)acrylic acid and 0.1 to 75 wt % AMPS® and 0.1 - 75 wt % alkyl(meth)acrylamide.
In the above examples, alkyl means C₁ to C₃₀, preferably C₁ to C₂₂, linear and branched and C₄ to C₁₆ cyclic; where (meth) is used, it means that the monomers with and without the methyl group are included. Other very useful polymers are swellable, but insoluble versions of poly(vinyl pyrrolidone) starch, carboxymethyl cellulose and polyvinyl alcohol.

Polymeric materials useful for the present purpose include (poly) hydroxyalkyl (meth)acrylate: anionic and cationic hydrogels: poly(electrolyte) complexes; Poly(vinyl alcohol) having a low acetate residual: a swellable mixture of crosslinked agar and crosslinked carboxymethyl cellulose: a swellable composition comprising methyl cellulose mixed with a sparingly crosslinked agar; a water swellable copolymer produced by a dispersion of finely divided copolymer of maleic anhydride with styrene, ethylene, propylene, or isobutylene; a water swellable polymer of N-vinyl lactams; swellable sodium salts of carboxymethyl cellulose.

Other gelable, fluid imbibing and retaining polymers useful for forming the hydrophillic hydrogel include pectin; polysaccharides such as agar, acacia, karaya, tragacenth, algins and guar and their crosslinked versions; Acrylic acid polymers, copolymers and its salt derivatives, polyacrylamides; polymer copolymers and their salt derivatives, water swellable indene maleic anhydride polymers; starch graft copolymers; acrylate type polymers and copolymers with water absorbability of about 2 to 400 times its original weight; diesters of polyglucan; a mixture of crosslinked poly(vinyl alcohol) and poly(N-vinyl-2-pyrrolidone).

Polyoxybutylene-polyethylene block copolymer gels, carob gum, polyester gels, poly urea gels, polyether gels, polyamide gels, polyimide gels, polypeptide gels, polyamino acid gels, poly cellulosic gels, Cyanamer®, crosslinked indene-maleic anhydride acrylate polymer, polysaccharides.

Representative polymers and the combinations of monomers possessing hydrophilic properties necessary to make synthetic hydrogel polymers are known to those skilled in the art. Some representative examples are disclosed in Scott et.al Handbook of Common Polymers CRC press Cleveland Ohio (1971).

Synthetic hydrogel polymers may be made by an infinite combination of several monomers in several ratios. In this instance the properties of the final hydrogel composition of this invention is the key parameter. The hydrogel can be crosslinked and it possesses the ability to imbibe and absorb fluid and swell or expand to an enlarged equilibrium state. The hydrogel is a polymeric composition and it swells or expands absorbing at least 2 to 1000 fold its weight of water. The preferred level of water absorption for such hydrogels is 2 to 500 fold its weight of water, whereas the most preferred range of water absorption is 3 to 100 times the weight of the dry polymer. Generally the optimum degree of swellability for a given hydrogel must be separately determined for different therapeutics depending upon their molecular weight, size, solubility and diffusively of each entrapped therapeutic and the specific spacing and cooperative chain motion associated with each individual polymer. For insulin immobilized within the acrylamide/acrylic acid copolymer of the invention crosslinked with ethylene N,N'-bis(acrylamide) as shown in Example 2, the swollen polymer has absorbed 10 - 20 times its weight of water. The polymers exhibits the ability to retain a significant fraction of imbibed fluid in the polymer molecular structure. The swellable hydrophillic polymers are also know as osmopolymers. The polymer can be of plant, animal, or synthetic origin provided that it can be crosslinked and the protease inhibitor can be functionalized in a manner where it is bound to the polymer hydrogel and cannot be separated from the polymeric hydrogel by washing.

The hydrophilic polymers useful in this invention are water insoluble but water swellable. It is convenient to refer to such water swollen polymers as hydrogels or gels. Such gels may be conveniently produced from water soluble polymer by the process of crosslinking the polymers by a suitable crosslinking agent. However, stable hydrogels may also be formed from specific polymers under defined conditions of pH, temperature and/or ionic concentration. It is required for this invention that a hydrogel stable under physiologically useful conditions be formed, regardless of the means by which its stability is achieved. Preferably the polymers are crosslinked, that is, crosslinked to the extent that the polymers possess good hydrophilic properties, have improved physical integrity (as compared to the non crosslinked polymers of the same or similar type) and exhibit improved ability to retain within the gel network both the enzyme inhibitor and the therapeutic material or materials, while retaining the ability to release the therapeutic material at the appropriate location and time.

Generally the hydrogel polymers should be crosslinked with a difunctional crosslinking in the amount of from 0.01 to 25 weight percent, based on the weight of the monomers forming the copolymer, and more preferably from 0.1 to 20 weight percent and more often from 0.1 to 15 weight percent of the crosslinking agent. Another useful amount of a crosslinking agent is 0.1 to 10 weight percent. Tri, tetra or higher multifunctional crosslinking agents may also be employed. When such reagents are utilized, lower amounts may be required to attain equivalent crosslinking density, i.e., the degree of crosslinking, or network properties which are sufficient to contain effectively the proteonase inhibitor.

The crosslinks can be covalent, ionic or hydrogen bonds with the polymer possessing the ability to swell in the presence of water containing fluids. Such crosslinkers and crosslinking reactions are known to those skilled in the art and in many cases are dependent upon the polymer system. Thus a crosslinked network may be formed by free radical copolymerization of unsaturated monomers. Polymeric hydrogels may also be formed by crosslinking preformed polymers by reacting functional groups found on the polymers such as alcohols, acids, amines with such groups as glyoxal, formaldehyde or glutaraldehyde, and bis anhydrides.

The polymers also may be crosslinked with any polyene, e.g. decadiene or trivinyl cyclohexane; acrylamides, such as N,N-methylene-bis (acrylamide); polyfunctional acrylates, such as trimethylol propane triacrylate; or polyfunctional vinylidene monomer containing at least 2 terminal CH₂ < groups, including, for example, divinyl benzene, divinyl naphthlene, and allyl acrylates. Particularly useful crosslinking monomers for use in preparing the copolymers are polyalkenyl polyethers having more than one alkenyl ether grouping per molecule. The most useful possess alkenyl groups in which an olefinic double bond is present attached to a terminal methylene grouping, CH₂=C<. They are made by the etherification of a polyhydric alcohol containing at least 2 carbon atoms and at least 2 hydroxyl groups. Compounds of this class may be produced by reacting an alkenyl halide, such as allyl chloride or allyl bromide, with a strongly alkaline aqueous solution of one or more polyhydric alcohols. The product may be a complex mixture of polyethers with varying numbers of ether groups. Analysis reveals the average number of ether groupings on each molecule. Efficiency of the polyether crosslinking agent increases with the number of potentially polymerizable groups on the molecule. It is preferred to utilize polyethers containing an average of two or more alkenyl ether groupings per molecule. Other crosslinking monomers include for example, diallyl esters, dimethallyl ethers, allyl or methallyl acrylates and acrylamides, tetravinyl silane, polyalkenyl methanes, diacrylates, and dimethacrylates, divinyl compounds such as divinyl benzene, polyallyl phosphate, diallyloxy compounds and phosphite esters and the like. Typical agents are allyl pentaerythritol, allyl sucrose, trimethylolpropane triacrylate, 1,6-hexanediol diacrylate, trimethylolpropane diallyl ether, pentaerythritol triacrylate, tetramethylene dimethacrylate, ethylene diacrylate, ethylene dimethacrylate, triethylene glycol and dimethacrylate. Allyl pentaerythritol, trimethylolpropane diallylether and allyl sucrose provide excellent polymers. When the crosslinking agent is present, the polymeric mixtures usually contain up to about 5 % or more by weight of crosslinking monomer based on the total of carboxylic acid monomer, plus other monomers, if present, and more preferably about 0.01 to 20 weight percent. A preferred crosslinking agent is alkylene N,N-bis(acrylamide), especially where the alkylene group is methylene or ethylene.

A critically important component in the composition of this invention is an enzyme inhibitor. It is generally well known that digestive enzymes which are found in the digestive tract, especially in the small intestines, rather quickly decompose proteins and peptides. Thus even if a therapeutically active protein or peptide is enclosed in a protective capsule or tablet which passes through the stomach unaffected and disintegrates in the intestines to release the therapeutically active ingredient, once that therapeutic is released it is immediately subject to the decomposing action of digestive (typically proteolytic) enzymes. The result is that after a relatively short period of time, such as even 30 minutes, there may be little of the therapeutic ingredient left in the intestines. It is precisely by this process that a protein is digested and decomposed into lower molecular weight components which provide nourishment. The bound inhibitor of proteolytic enzymes incorporated in the composition of this invention helps to protect the therapeutically active protein or peptide from the action of the proteolytic enzyme and increases the probability that a therapeutic will be absorbed by the mucosa of the intestines. It is due at least partially to the action of proteolytic enzymes that when a protein or peptide is delivered through the stomach into the intestines it is relatively quickly digested and, therefore, cannot be effectively absorbed into the bloodstream.

Any inhibitor which inhibits the activity of proteolytic enzymes may be usefully employed in the composition and the delivery system for proteins and peptides. Useful inhibitors include soybean trypsin inhibitor, pancreatic trypsin inhibitor, chymotrypsin inhibitor and trypsin and chrymotrypsin inhibitor isolated from potato (solanum tuberosum L.) tubers (see Isolation and characterization of a novel trypsin and chymotrypsin inhibitor from potato tubers. Revina, T.A.; Valueva, T.A.; Ermolova, N.V.; Kladnitskaya, G.V.; Mosolov, V.V. A.N. Bakh Inst. Biochem., Russian Academy Sci., Moscow, 117071, Russia. Biokhimiya (Moscow) (1995), 60(11), 1844-52. CODEN: BIOHAO; ISSN: 0320-9725. Journal written in Russian. CAN 124:49041). A combination or mixtures of inhibitors may be employed.

The most preferred and desirable inhibitor of said proteolytic enzymes is an ovomucoid-enzyme which may be isolated from the white of duck eggs, turkey eggs (chicken eggs or the eggs of other fowl) (cf. USSR Inventor's Certificate N 1404513, IPC C07 K 3/02.B.I. I 23 1988), or which may be obtained by conventional biosynthesis, separation and purification approaches, genetic engineering, or other appropriate methodologies which permit the enzyme to be isolated, collected, and purified. The most preferred inhibitor is the ovomucoid enzyme isolated from the white of the duck eggs. Other inhibitors and hydrogel functionalizing agents may be appropriate for protecting enzymes from other mechanisms of degradation depending upon the specific protein used, its susceptibility to degradation under specific conditions, and the target barrier for transmission into the circulatory system.

Protecting a bioactive protein from the action of the proteolytic enzymes by the use of appropriate inhibitors is a critically important feature of this invention. The useful inhibitors should contain a sugar (glycoside) moiety, and if an inhibitor is devoid thereof, such moieties may be introduced by covalently bonding them onto the hydrogel polymer or through a linking agent such as the inhibitor itself. It is important that the protective agent be retained within the structure of the hydrogel. This is why one critical aspect hydrogel. This effect is illustrated in the subsequent examples. The inhibitor may also be immobilized through sufficient physical entrapment within the polymer matrix. However, by itself even this would not result in a truly successful and effective delivery system. It is further necessary to increase the association of the protein or peptide-containing polymer hydrogel with selected transport barriers of the intestine and/or bowels to facilitate access and transport of the therapeutic substances through the barriers and into the circulatory system. This may be accomplished by incorporating into the composition (and the delivery system) of this invention a functional agent such as, but not limited to, a glycoside, a sugar containing molecule or a binding agent such as a lectin binding agent which is known to interact with the desired intestinal transport barrier. Again, it is necessary that this associative agent or functionality be bound to the hydrogel. In this manner the hydrogel preferably attaches itself to the intestinal walls and facilitates the passage of the therapeutic protein or peptide into the circulatory system. If the associative agent is not bound, it may easily diffuse away from the formulation and its effect will be lost. This effect is also illustrated in the subsequent examples.

The functional agents such as glycosides may be chemically incorporated into the polymeric hydrogel. Examples of such glycosides are glucosides, fructosides, galactosides, arabinosides, mannosides and their alkyl substituted derivatives and natural glycosides such as arbutin, phlorizin, amygdalin, digitonin, saponin, and indican. There are several ways in which a typical glycoside may be bound to polymer. For example, the hydrogen of the hydroxyl groups of a glycoside or other similar carbohydrate may be replaced by the alkyl group from the hydrogel to form an ether. Also, the hydroxyl groups of the glycosides may be reacted to esterify the carboxyl groups of the polymeric hydrogel to form polymeric esters in situ. Another approach would be to employ condensation of acetobromoglucose with cholest-5-en-3.beta.-ol on a copolymer of maleic acid. N-substituted polyacrylamides can be synthesized by the reaction of activated polymers with omega-aminoalkylglycosides: (1) (carbohydmte-spacer)(n)-polyacrylamide, 'pseudopolysaccharides'; (2) (carbohydrate spacer)(n)-phosphatidylethanolamine(m)-polyacrylamide, neoglycolipids, derivatives of phosphatidylethanolamine; (3) (carbohydrate-spacer)(n)-biotin(m)-polyacrylamide. These biotinylated derivatives may attach to lectins on the gut wall and facilitate absorption of encapsulated proteins and peptides.

Glycoproteins having N,N'-diacetylchitobiose and N-acetyllactosamine can be synthesized on the basis of radical copolymerization of n-pentenylated derivatives with acrylamides (see Macromolecules (1991), 24(15), 4236-51 and On Tai Leung *et al* New J. Chem (1994), 18(3), 349-63).

It is possible and indeed most preferable that the proteolytic enzyme inhibitor also contain the associative binding agent. For this reason ovomucoid, which is a specific example of a class of glycoproteins, is the most preferred proteolytic enzyme inhibitor. It has a molecular weight of about 31000. The amino-acid composition of the protein portion of ovomucoid is given in Table I. A molecule.of ovomucoid comprises 12.5% by weight of glucosamine and 7.8 % by weight of other sugars. It is known that the villi and other structures of the intestine mucosa contain lectin (sugar) bearing receptors. Hence, the presence of lectin-like groups attached to the gel matrix is a useful aspect of the invention. While the gel material may be separately functionalized to contain lectin-like functionality and an enzyme inhibitor, a bound ovomucoid molecule incorporates both functionalities and, as may be seen from the examples, gives optimal enhancement for the delivery of incorporated insulin. Of the various ovomucoids which may be utilized, ovomucoid from duck eggs contains 3 active centers which may bind a wide variety of enzymes. It is the most preferred ovomucoid. ovomucoid derived from chicken and turkey eggs have fewer binding centers. When a lectin containing enzyme inhibitor is used for the delivery of insulin, it is preferred that in the protective enzyme inhibitor the sugar content should be at least 3%, although a lower content may be acceptable for some applications and the delivery of some therapeutics under certain circumstances. It is an important aspect of the invention that the enzyme inhibitor, whether ovomucoid or some other glycoprotein serving a similar function, contains both an interactive functionality for the target transport barrier, and the chemical and conformational characteristics necessary to protect the therapeutic protein with which it is closely intermixed, and /or associated within the hydrogel. It is further taught and is an important aspect of the invention that the binding functionality whether a lectin binding agent or some other agent, may be separately incorporated and functionalized into the polymer of the hydrogel, the protective enzyme inhibitor then being separately bound or immobilized to the polymeric matrix material.

The amount of the inhibitor, that is, the proteolytic enzyme inhibitor containing also a lectin binding agent, that needs to be incorporated in the composition of this invention will vary depending on (a) the specific inhibitor (e.g., the source of the ovomucoid, such as from the duck eggs, turkey eggs, chicken eggs or eggs of other fowl or from potato tubers), (b) the number of functional groups present in the molecule which may be reacted to introduce ethylenic unsaturation necessary for copolymerization with the hydrogel forming monomers, and (c) the number of lectin groups, such as glycosides, which are present in the inhibitor molecule. It may also depend on the specific therapeutic protein which is intended to be introduced into the circulatory system. Generally speaking, the amount should be sufficient to provide the desired proteolytic enzyme inhibiting function (protection of the therapeutic) and the desired binding action with the intestinal receptors. It may be necessary to conduct some routine tests to determine the optimum amount but this would be evident to those skilled in the art.

A useful amount of an ovomucoid is from 0.1 mg per ml. of gel to 50 mg per ml. of gel and preferable from 0.2 mg per ml of gel to 25 mg per ml of gel. The above preferred range is especially applicable when ovomucoid isolated from the duck eggs is used. However, use of ovomucoid in an amount greater than 25 mg per ml of gel causes no particular harm, and may provide greater shelf life or long term stability of the preformulated materials. In addition, larger concentrations of ovomucoid may provide added benefit in terms of longer term release of some materials.

When the inhibitor function and the binding action function are incorporated or bound to the polymer hydrogel separately (i.e., they are not part of the same molecule as is in the case of an ovomucoid), the amount of each ingredient that must be bound to the polymer will be determined in a similar manner as described above.

Ovomucoid is representative and the most important proteolytic enzyme inhibitor of the invention. Ovomucoid isolated from duck egg white is a glycoprotein containing 12.5 % glucosamine (21.6 mole per mole protein) and 7.8% other sugars, giving a reaction with phenol sulphuric acid.

The molecular mass of the ovomucoid determined by the light-scattering method is 30 900 ± 400, close to the values calculated from amino acid analysis and carbohydrate composition (31 500) and determined by polyacrylamide gel electrophoresis in the presence of sodium dodecyl sulphate and β-mercaptoethanol (30 500 ± 1000). Its composition is given in Table 1.

**TABLE 1**

| AMINO-ACID CONTENT OF OVOMUCOID FROM DUCK EGG WHITE | |
|---|---|
| AMINO ACID | RESIDUES PER MOLECULE |
| Asp | 30.14(30) |
| Thr | 17.98(18) |
| Ser | 12.98(13) |
| Glu | 23.63(24) |
| Pro | 10.24(10) |
| Gly | 19.75(20) |
| Ala | 10.45(10) |
| ½ Cys | 13.56(14) |
| Val | 14.77(15) |
| Met | 6.32 (6) |
| Ile | 3.59 (2) |
| Leu | 12.76(13) |
| Tyr | 9.53(10) |
| Phe | 5.00 (5) |
| Lys | 16.36(16) |
| His | 4.23 (4) |
| Arg | 1.95 (2) |
| Trp | 0 |
| | |
| Total | (212) |
| Mᵣ Residues | 25,178.0 |

For the composition of this invention to be effective as an oral delivery system for the therapeutic proteins and peptides, it is necessary to prepare the composition in a particular way and not simply admix the necessary ingredients. As noted above, one important function of the proteolytic enzyme inhibitor is to protect the protein or the peptide from decomposition by the enzyme. Ovomucoid is known to be an inhibitor of proteolysis, but if a protein and ovomucoid are simply admixed and, for example, placed into water, both will diffuse and ovomucoid will not be able to protect the protein from degradation. Such formulations will not be useful as interactive delivery systems to deliver a therapeutic across the intestinal barrier. Thus an important aspect of this invention is the specific combination of the proteolytic enzyme inhibitor, and a binding agent with a polymeric hydrogel. This is accomplished by chemically modifying the polymer hydrogel with a material which contains both a proteolytic inhibitor and a binding agent (e.g., ovomucoid) immobilizing the inhibitor and the binding agent by making it part of the crosslinked polymer network. It is also possible to chemically modify the gel separately with an interactive agent such as a glycosidal containing molecule, and a nonglycosidal containing proteolytic enzyme inhibitor.

Generally speaking, first the inhibitor is functionalized, that is, an appropriate reactive group is chemically added to the inhibitor, most often an ethylenic polymerizable group is added, and the functionalized inhibitor is then copolymerized with monomers and a crosslinking agent using a standard polymerization method such as solution polymerization (usually in water), emulsion, suspension or dispersion polymerization. More specifically, the functionalization agent should have a high enough concentration of functional or polymerizable groups to insure that several sites on the inhibitor are functionalized. For example, in ovomucoid there are thirteen amine sites which may be functionalized and it is preferred for this invention that at least five of such sites are functionalized. After functionalization, the functionalized inhibitor is mixed with the monomers and a crosslinking agent which comprise the reagents from which the polymer gel is formed. Polymerization is then induced in this medium to create a polymeric gel containing the bound inhibitor. The gel is then washed with water or other appropriate solvents and otherwise purified to remove trace unreacted impurities and, if necessary, ground or broken up by physical means such as by stirring, forcing it through a mesh, ultrasonication or other suitable means to the preferred particle size. The solvent, usually water, is then removed in such a manner as to not denature the inhibitor. The preferred method is lyophillization (freeze drying) but other methods are available and may be used (e.g., vacuum drying, air drying, spray drying, etc.). A more detailed description of the above described method is given in Example 3.

In functionalizing the inhibitor protein the object is to introduce a functional group onto the surface of the inhibitor protein that can subsequently react in polymerization or otherwise couple the inhibitor protein to the hydrogel. As examples of introducing polymerizable groups one may react available amino, hydroxyl and thiol groups from the protein inhibitor, with electrophiles containing unsaturated groups. For example, unsaturated monomers containing N-hydroxy succinimidyl groups, active carbonates such as p-nitrophenyl carbonate, trichlorophenyl carbonates, tresylate, oxycarbonylimidazoles, epoxide, isocyanates and aldehyde, and unsaturated carboxymethyl azides and unsaturated orthopyridyl-disulfide belong to this category of reagents. Illustrative examples of unsaturated reagents are allygylcidyl ether, allyl chloride, allylbromide, allyl iodide, acryloyl chloride, allyl isocyanate, allylsulfonyl chloride, maleic anhydride, copolymers of maleic anhydride and allyl ether.

All of the lysine active derivatives, except aldehyde, can generally react with other amino acids such as imidazole groups of histidine and hydroxyl groups of tyrosine and the thiol groups of cystine if the local environment enhances nucleophilicity of these groups. Aldehyde containing functionalizing reagents are specific to lysine. These types of reactions with available groups from lysines, cystines, tyrosine have been extensively documented in the literature and are known to those skilled in the art.

In the case of ovomucoid which contains amine groups, it is convenient to react such groups with an acyloyl chloride, such as acryloyl chloride, and introduce the polymerizable acrylic group onto the ovomucoid. Then during the preparation of the polymeric hydrogel, such as during the crosslinking of the copolymer of acrylamide and acrylic acid, the functionalized ovomucoid, through the acrylic groups, is attached to the polymer and becomes bound thereto.

The therapeutic ingredients which may be administered orally employing the delivery system and the compositions of this invention are proteins and peptides including monopeptides, dipeptides, tripeptides, and polypeptides. Also may be administered orally proteins which are modified to act as carriers for biologically active materials, including anti-sense oligonucleotides, genes and viral vector drug carriers and many conventional organic compounds of low stability, solubility and consequently low oral bioavailability.

Illustrative examples of therapeutic proteins are nucleoprotein, glycoprotein, lipoprotein, immunotherapeutic, porcine somatotropin for increasing feed conversion efficiency in a pig, growth hormone, buserelin, leuprolide, interferon, gonadorelin, olamine, calcitonin, and other bioactive proteins and recombinant protein products.

Illustrative examples of peptides are cyclosporin, lisinopril, captopril, delapril, cimetidine, ranitidine, famotidine (Pepcid), tissue plasminogen activator, epidermal growth factor, fibroblast growth factor (acidic or basic), platelet derived growth factor, transforming growth factor (alpha or beta), vasoactive intestinal peptide, tumor necrosis factor; hormones such as glucagon, calcitonin, adrecosticotrophic hormone, aldoetecone, follicle stimulating hormone, enkaphalins, β-endorphin, somatostin, gonado trophine, α-melanocyte stimulating hormone. Additional examples are bombesin, atrial natriuretic peptides and luteinizing hormone releasing (LHRII), substance P, vasopressin, α-globulins, transferrin, fibrinogen, β-lipoproteins, β-globulins, prothrombin (bovine), ceruloplasmin, α₂-glycoproteins, α₂-globulins, fetuin (bovine), α₁-ligoproteins, α₁-globulins, albumin and prealbumin.

The growth hormones which may be delivered orally using the invention may be commercially available human growth hormone, bovine growth hormone, porcine growth hormone, or other representative animal growth hormone extracted from natural sources, or produced via genetic engineering and related production fermentation methodologies which are well known and described in the art of protein and peptide manufacture.

Proteins that may be administered orally employing the composition of this invention should be of molecular weight less than 1,000,000 daltons, and preferably less than 200,000. More usually the molecular weight of proteins less than 65,000 and often under 32,500 and especially less than 10,000 daltons. Usually they have a molecular weight of at least 300 daltons.

The composition of this invention, that is, a composition adapted for the oral administration of proteins or peptides other than insulin comprising a polymeric hydrogel which, preferably, has been crosslinked, and which has been modified by a proteolytic enzyme inhibitor and a binding agent, and contains a protein or a peptide, may be administered in a gel form. Generally any desired dose may be administered to a human or an animal. The dose actually delivered can be calculated based on the amount of the biologically active therapeutic material contained in a unit weight or volume of the gel. However, when a gel containing non-bound therapeutic is introduced to the stomach which contains no free therapeutic there is a kinetic driving force which must result in rapid loss of the therapeutic from the geL For example, insulin containing hydrogel placed in a pH adjusted saline loses 90% of its contained insulin in 15 minutes. Unless rapid and reproducible dumping of the stomach contents into the intestine can be assured, variable, and unreliable dosages will be delivered from patient to patient, and from one administration to the next, making controllable therapeutic and biological effects difficult to assure.

It is, therefore, more convenient and preferred to administer the therapeutic containing composition of this invention in tablet form or gel capsules as is well known to the art. When administered in that form then the tablet or gel capsule is preferably enterically coated. The enteric coating assures that the therapeutic-containing composition passes into the intestinal tract without being significantly affected or partially degraded in the stomach, regardless of the time profile of delivery to the intestine. In the intestinal tract the coating dissolves and the composition functions as described above.

Enteric coating materials useful in the present invention include those coating materials resistant to degradation in the stomach but which will decompose in the environment of the intestinal tract to expose the coated material. Such enteric coating materials included, the following ingredients either singly or in combination: hydroxypropyl methylcellulose phthalate, polyethylene glycol-6000, and shellac, and they may be dissolved in solvents including dichloromethane, ethanol and water, cellulose phthalate, or polyvinyl acetate phthalate.

A useful enteric coating material for coating insulin comprises 4.5 parts by weight hydroxymethylcellulose to 0.5 parts shellac, to 0.5 parts polyethylene glycol-6000. This material is dissolved in 47.3 parts dichloromethane and 37.8 parts ethanol. The enteric coating material is then diluted with water to an optimal concentration and is applied to the compositions of the invention.

Many other coating materials useful for pharmaceutical preparations are well known and available in the industry. They include conversion products of polysuccinimide as disclosed in U.S. Patent 5,175,285; acrylic polymers such as those derived from lower alkyl esters of acrylic and methacrylic acids which may be copolymerized with other copolymerizable monomers, such as those containing carboxyl or quaternary ammonium groups and/or with acrylic or methacrylic acid as described in U.S. Patent 5,422,121 and U.S. Patent 4,644,031.

Other well known ingredients may also be incorporated. Examples are binder materials, such as various types of cellulose and their derivatives, providone and polyethylene glycol fatty acid ester groups; fillers, such as gum acacia, crystalline cellulose, hydroxypropylcellulose and hydroxypropylmethylcellulose; surfactant materials, such as cationic anionic or nonionic surfactants illustrated by sodium lauryl sulfate, stearyl amine, polyglycerine fatty acid esters and many other fatty acids, fatty acid esters, and emulsifying agents, such as cholesterol, stearic, palmic and oleic acids and their sodium salts, salicylic acids, salts or esters, various stearates and polysorbates (20, 40, 60, 80) propylene glycol diacetate; and antimicrobiol agents, such as methylparaben, ethylparaben, propylparaben, butylparaben, phenol, dehydroacetic acid, phenylethyl alcohol, sodium benzoate, sorbic acid, thymol, thimerosal, sodium dehydroacetate, benzyl alcohol, butylparaben, cresol, p-chloro-m-cresol, chlorobutanol, phenylmercuric acetate, phenylmercuric borate, phenylmercuric nitrate and benzalkonium chloride.

The invention will be further understood and illustrated by referring to the following examples.

### Example 1

### Isolation of Ovomucoid

To a flask equipped with a stirrer was added 100 g of egg white. Next 100 g of a mixture comprised of 70 g of ethyl alcohol, 30 g of a solution comprised of 2.7 g trichloracetic acid and 27.3 g of distilled water was added to the flask at a rate of from 3 to 5 ml/min while stirring. Upon addition of the entire amount of the specified mixture the reaction mass was further stirred for 40 min. at a temperature which may range between 15 and 25°C. The precipitate was filtered, and removed from the remaining solution. This precipitate was then rejected having no further value. The filtrate from this step was then transferred to a second flask and mixed with 550 g of ethyl alcohol. The precipitate from this latter step was then dialyzed using distilled water and then dried by lyophilization. The yield from this procedure was about 0.8 g of ovomucoid.

The above procedure is specific to the isolation of ovomucoid from duck eggs. However, the same procedure may be used to obtain ovomucoid from other sources using the egg white of other fowl, or other protein and glycoprotein containing sources replacing the 100 gm of egg white used in step one above. More or less ovomucoid may be obtained depending on the source material, and the variability of ovomucoid in the source material. In addition, the conditions of extraction and precipitation may be varied in temperature and time, and the quantities and types of solvents may also be varied to optimize the extraction of ovomucoid from different materials.

### Example 2

### Functionalization of Ovomucoid

In a 20 ml beaker, 10 ml of a NaHCO₃ buffer solution at pH 8 was stirred. Isolated ovomucoid (100 mg) was added and dissolved with agitation. Acryloyl chloride (10 µl) was added to this solution at room temperature and stirred until the reaction was complete.

### Example 3

### Preparation of Polymer

To the solution obtained in Example 2 was added acrylamide (1 g) [when commercial grade acrylamide is used it contains up to about 1.5% of acrylic acid] and N,N-methylene-bis (acrylamide) (0.1 gm). To this solution 10 mg of (NH₄)₂S₂O₈ and 10 µl of TMEDA (tetramethylethylene diamine) were added to initiate polymerization. The reaction mixture was stirred to insure good mixing but stopped just before the cloud point of polymerization. The resulting gel was screened through a nylon mesh. The particles were washed with NaHCO₃ buffer solution and then lyophylized until dry.

### Example 4 (Reference)

### General Procedure for Preparing Orally Active Insulin Composition

For insulin therapeutics a representative composition is prepared in the following manner: 0.01 - 1.0 g of a pre-dried 4.5 % crosslinked polymer modified with 0.2 - 25 mg of soybean trypsin inhibitor per ml of swollen gel and 0.2 - 25 mg of a gylcoside per ml of swollen gel is placed in 0.3 - 3.0 ml of insulin solution (conveniently in a buffered saline solution) with insulin being at a concentration of 0.01 to 3 mg/ml of liquid (insulin activity may range from 0.25 - 100 I.U./ml for 1 hour at room temperature. During this time the polymer swells completely, imbibing the insulin containing solution, and reaching equilibrium and is ready for use. This methodology for preparation is not limited to the specific time given, and may be judiciously varied over a range of times and temperatures depending on a number of variables such as the crosslink density of the polymer, the temperature under which the exposure of hydrogel is carried out. The polymer may also be exposed to larger or smaller amounts of insulin concentration, and/or amounts of fluid, and of course the total amount of material processed may be increased or decreased by changing the amount of insulin containing solution and hydrogel used in the process.

Following are the polymers used in the following Examples and the tables as reported in Tables 2 to 5. These crosslinked hydrogel polymers were prepared according to the procedure of Example 3.
- PAA -: polyacrylamide
- PMA -: polymethacrylamide
- PHEMA -: polyhydroxyethylmethacrylate
- PVP -: polyvinylpyrrolidone
- PAAC -: polyacrylic acid

### EXAMPLE 5

### General Procedure for Preparing and Administering Oral Formulations

The polymer was prepared according to Example 3 using the crosslinking agent in the amount of 0.5 % of the initial monomer solution. 0.1 g of a pre-dried crosslinked polyacrylamide polymer (containing possibly up to 3 % by weight of acrylic acid) modified with ovomucoid (isolated from the white of duck eggs except when indicated otherwise) is placed in an excess of aqueous growth hormone solution in the concentration of 1.0 mg/ml of the saline solution at room temperature. The polymer is exposed to the solution for a period of 1 hour. After this period of time the imbibing of the growth hormone containing solution reaches equilibrium and the polymer has swollen completely. The polymers that show a measurable effect will have absorbed at least 0.5 g of aqueous solution of the growth hormone. The formulation was removed from the growth hormone solution and was ready for use.

### EXAMPLES 6 - 7

### ORAL GROWTH HORMONE DELIVERY

An unmodified polyacrylamide (PAA) gel sample is caused to imbibe approximately 100 counts of Radioactive labeled human growth hormone (HGH) intermixed with a normal therapeutic dosage of growth hormone (10 µg/kg). The methodology for absorption is given in Example 5. The sample counts are determined and the sample is administered orally to rats as a control (Example 6). No count of growth hormone is observed in the blood by radioimmunoassay after 2 hours. Another sample of PAA modified with ovomucoid as described in Example 3 is similarly treated, measured and administered. Approximately one hundred counts are determined to be present in the sample prior to administration. After 2 hours, approximately 25 counts can be measured as having passed through the intestinal barrier and entered the circulatory system using the same radioimmunoassay techniques as used in Example 6. The results are shown in Table 2.

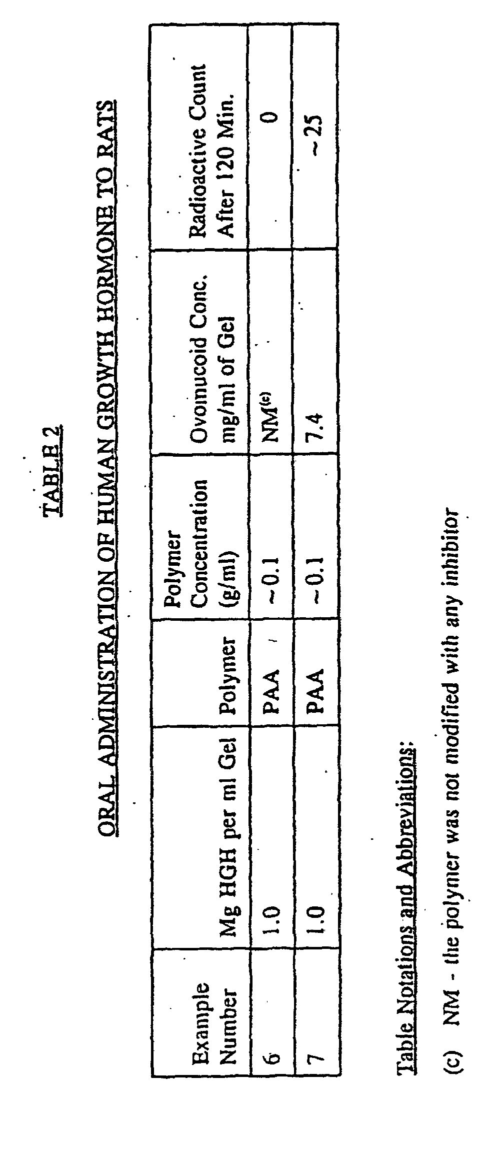

It is clearly seen from data in Table 2 that the formulation of the claimed invention, that is, where an inhibitor of proteolytic enzymes was chemically bonded to the polymer matrix, has the ability to prevent growth hormone degradation in the gastrointestinal tract and enables the passage of a significantly efficacious quantity of growth hormone into the host animals bloodstream.

### EXAMPLE 8

The protective effect of ovomucoid on proteins ranging in molecular weight from 16,000 to well in excess of 300,000 dalton is shown in Table 3. In the experiments reported in Table 3, trypsin, a proteolytic enzyme, was incorporated in the indicated molar ratio with the protein to simulate the physiological condition in the intestinal tract where a lytic enzyme initiates the hydrolysis of the protein. It can clearly be seen that a very small quantity of the inhibitor can protect a large amount of protein (in excess of 300 times its molar amount) against proteolytic degradation. It is easily seen that while more than 90 percent of the protein remains undergraded in the presence of a protective inhibitor, less than one percent is found to remain after only 2 hours when no ovomucoid inhibitor is present. The effect of incorporating ovomucoid is to enhance the active protein present at physiologically meaningful times by a factor of more than 10, and for many of the materials studied by approximately 100 or more over the two hour period following ingestion.

**TABLE 3**

| PROTECTIVE EFFECT OF OVOMUCOID^{(a)} | | | | |
|---|---|---|---|---|
| Protein | Molecular Weight | Molar Ratio Protein/Enzyme | Ovomucoid Inhibitor (mg) | Degradation at 2 Hr. 25°C in Water |
| Myoglobin | 17,000 | 300 / 1 | 1.0 mg | 4 - 5% |
| Myoglobin | 17,000 | 300 / 1 | 0.0 mg | >99% |
| Ovalbumin | 43,000 | 300 / 1 | 1.0 mg | 4 - 5% |
| Ovalbumin | 43,000 | 300 / 1 | 0.0 mg | >99% |
| Serum Albumin | 69,000 | 300 / 1 | 1.0 mg | 6 - 7% |
| Serum Albumin | 69,000 | 300 / 1 | 0.0 mg | >99% |
| Fibrinogen | 340,000 | 300 / 1 | 1.0 mg | 6 - 7% |
| Fibrinogen | 340,000 | 300 / 1 | 0.0 mg | >99% |

| | | | | |
|---|---|---|---|---|
| (a) The proteolytic enzyme is Trypsin The amount of Trypsin 0.5 mg | | | | |

It was pointed out above that the protease inhibitor must be chemically bound (immobilized) to the hydrogel polymer. Another method of binding (immobilizing) the protease inhibitor into the hydrogel network is to entrap the protease inhibitor in the network via physical means rather than covalent bonding. In this manner the network that is formed via crosslinking is such that the protease inhibitor is trapped within the mesh structure due to its three dimensional structure being larger than the three dimensional openings of the hydrogel network. Other methods of association such as hydrogen bonding, salt association, and hydrophobic association that will cause retention of the protease inhibitor within the hydrogel matrix are known to those skilled in the art and may also be employed in this invention to bind (immobilize).

The protective effect of ovomucoid on proteins ranging in molecular weight from 16,000 to over 300,000 dalton is shown in Table 4. In the reported experiments α-chymotrypsin, a proteolytic enzyme, was incubated in the indicated molar ratio with the protein to simulate the physiological condition in the intestinal tract where a proteolytic enzyme initiates the hydrolysis of the protein. Two hydrogels were prepared. One containing ovomucoid-containing gel prepared as described in example 3 and one prepared without ovomucoid. 2.5 mg protein per ml swollen hydrogel was introduced into the swollen hydrogel and then α-chymotrypsin was added. The protein: α-chymotrypsin ratio was 200:1. The protein/hydrogel/α-chymotrypsin solution was incubated for 1 hr at 30°C in a phosphate buffer (pH 7.4). The products of proteolysis were washed from the hydrogel using phosphate buffer (10x volume). The degree of hydrolysis was estimated by HPLC to show the amount of degraded protein vs. non-degraded protein.

From Table 4, it is easily seen that more than 90% of the protein remains undegraded in the presence of the protective hydrogel. Without the protective gel environment, over 90% of the protein is degraded. The effect of incorporating ovomucoid is to enhance the active protein present at physiologically meaningful time by a factor of more than 10, and for many of the materials studied by approximately 100 or more over the one hour period following ingestion.

**TABLE 4**

| PROTEIN/PEPTIDE HYDROLYSIS WITH CHYMOTRYPSIN | | | |
|---|---|---|---|
| Protein/Peptide in Hydrogel | MW of Protein/Peptide | Degree of Hydrolysis, % | |
| | | I | II |
| | | | |
| Ribonuclease A | 13700 | ≥90 | ≤5 |
| Myoglobin | 17800 | ≥90 | ∼5 |
| Ovalbumin | 45000 | ≥90 | ∼5 |
| Bovine serum albumin | 68000 | ≥90 | ∼5 |
| Aldolase | 158000 | ≥95 | 5-10 |
| Catalase | 232000 | ≥95 | 5-10 |
| Fibrinogen | 340000 | ≥95 | 5-10 |
| Ferritin | 440000 | ≥95 | ∼10 |
| Thyroglobulin | 669000 | ≥95 | ∼10 |
| I - protein physically immobilized in norunodified polyacrylamide hydrogel; II - protein physically immobilized in ovomucoid-modified polyacrylamide hydrogel. | | | |

Tables 5 and 6 show that gel formulations within the invention can effectively deliver protein and peptide drugs. For example, Table 5 shows that an ovomucoid modified polyacrylamide hydrogel administered orally can deliver effectively a dosage level of Glucagon which, currently must be delivered by subcutaneous injection. Glucagon is a 29-member low molecular weight protein molecule which is known to cause a dramatic increase in glucose level upon release into the blood stream. It accomplishes this by stimulating the splitting of glycogen in the liver. Because of this effect it is useful in treating certain forms of hypoglycemia. Since it is a protein, it cannot be effectively delivered orally. Table 6 shows the effect of administering calcitonin using oral dosage forms of the invention. Rabbits are the test animals in both cases. In both cases the specified amount of active protein drug is admixed with the gel as described above. A solution is prepared of the drug in water. pH may be adjusted to improve the stability or other properties of the drug, but this is not necessary. The solution is then added to the dry polymer in the amount of 1 ml of drug containing solution to 100 mg of dry polymer, though other amounts of dry polymer may be used (e.g. 50 mg per ml of solution).

Table 5 shows that simply giving the animal physiological saline or even a normal subcutaneous injection dosage of glucagon in solution as an oral gavage has no effect on blood sugar. In both eases, blood sugar remains constant and at normal levels through the time course of the experiment. However, giving the same dosage in the form of a subcutaneous injection causes a precipitous rise in blood sugar. Glucagon measured in the blood shown to increase upon injection of this amount of glucagon. Finally, Table 5 clearly shows that when the same amount of glucagon (0.5 mg) is administered to rabbits with one of the active formulations of the invention, (in this case ovomucoid modified polyacrylamide hydrogel) blood sugar and blood glucagon levels rise in a manner very similar to that observed in the case of the subcutaneous injection control. Clearly, the formulation is active in enhancing the oral delivery of glucagon.

Table 6 shows the effect of giving a standard injection therapeutic dose of calcitonin by oral means. Calcitonin is a low molecular weight protein currently administered by injection, is known to increase turnover of Calcium and may be effective in treating a number of diseases involving calcium such as bone embrittlement, diseases of the thyroid gland, and other diseases in which calcium is known to play a vital role. Table 6 illustrates that a single dose of calcitonin given orally with a gel formulation containing immobilized ovomucoid has approximately the same effect after 60 minutes as a subcutaneous injection.

Table 6 shows data of the mmol of Ca²⁺/1 of blood in a rabbit after 1 mg of calcitorin was administered subutaneously and orally in a polyacrylamide gel which had been modified with ovomucoid isolated from the duck egg whites. Procedures described above were used for the preparation of the modified polymers and for absorbing calcitonin into the gel. The concentration of calcium in the blood was determined by a standard procedure using selective electrode. The data confirms that calcitonin is protected by the ovomucoid-modified hydrogel when it is administered orally.

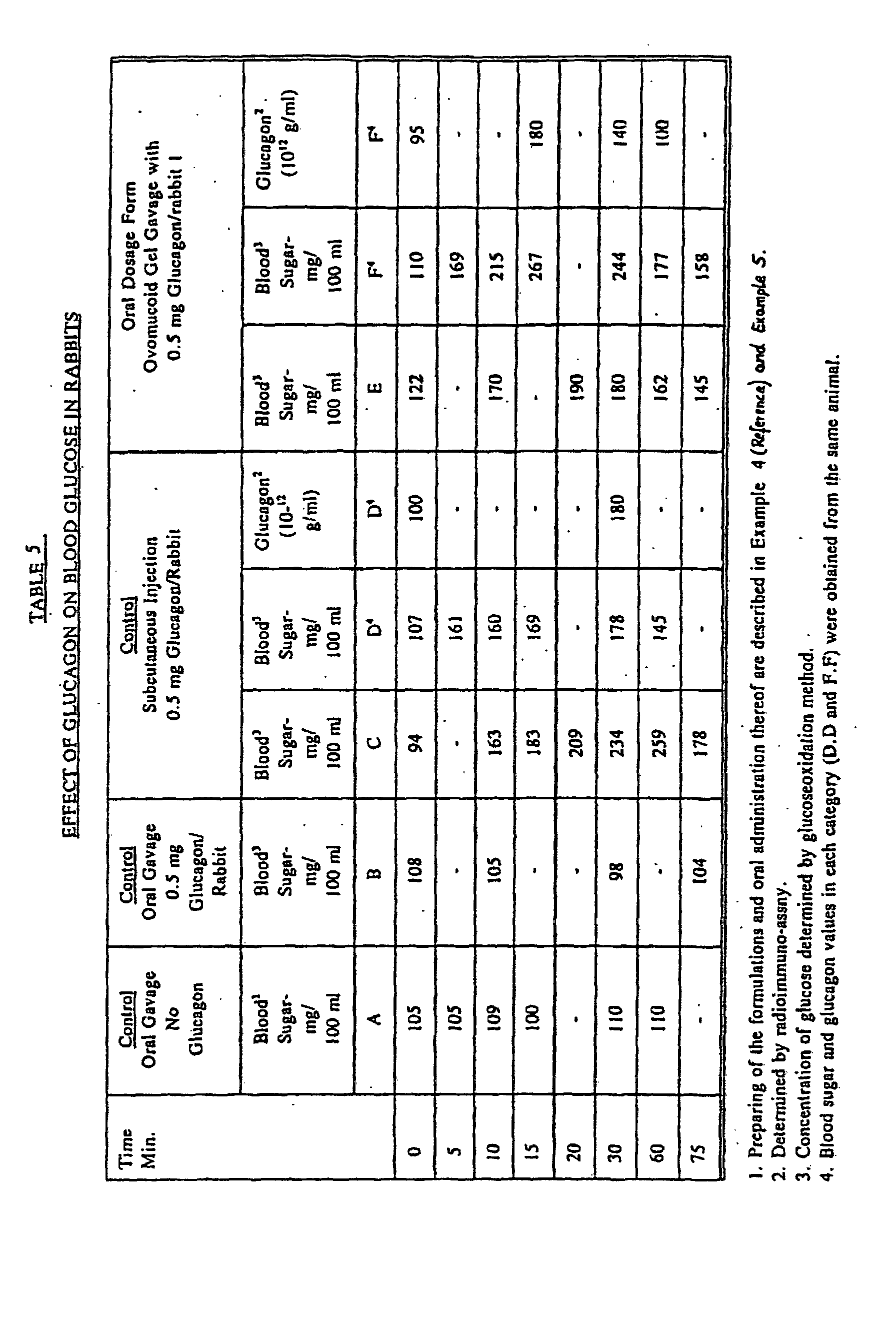

**TABLE 6**

| Time Min | Control Subcutaneous 1 mg Calcitonin / Rabbit | Oral Ovomuicoid Gel Gavage 1 mg Calcitonin/Rabbit¹ |
|---|---|---|
| | (mmole/Ca²⁺/l) | (mmole/Ca²⁺/l) |
| 0 | 1.09 | 0.99 |
| 30 | 1.20 | 1.02 |
| 60 | 1.39 | 1.42 |

| | | |
|---|---|---|
| 1. Preparation of the formulations and oral administration thereof are described in Example 4 (Reference) and Example 5. | | |

The below listed proteins/peptides were tested as described below to determine the level of transport across the polyacrylamide and (Concanavalin A) Con-A-containing polyacrylamide hydrogel. The results are reported in Tables 8-13.

**TABLE 7**

| PROTEIN AND PEPTIDES | | |
|---|---|---|
| | Protein/Peptide | MW of Prot./Peptide (10g MW) |
| 1 | Calcitonin | 3400 (3.53) |
| 2 | Glucagon | 3500 (3.54) |
| 3 | Insulin (Reference) | 6500 (3.81) |
| 4 | Ribonuclease A | 13700 (4.14) |
| 5 | Myoglobin | 17800 (4.25) |
| 6 | Trypsin inhibitor from soybean | 21500 (4.33) |
| 7 | Chymotrypsinogen A | 25000 (4.40) |
| 8 | Ovalbumin | 45000 (4.65) |
| 9 | Bovine serum albumin | 68000 (4.83) |
| 10 | Aldolase | 158000 (5.20) |
| 11 | Catalase | 232000 (5.37) |
| 12 | Fibrinogen | 340000 (5.53) |
| 13 | Ferritin | 440000 (5.64) |
| 14 | Thyroglobulin | 669000 (5.83) |

Polyacrylamide hydrogels were synthesized by polymerizing aqueous solutions of acrylamide (10%) and N,N'-methylenebisacrylamide (0.5%) under the action of redox catalyst - ammonium persulfate - N,N,N'N'-tetramethylethylenediamine. Modified hydrogels were prepared by polymerization of the same monomer mixture in the presence of 1.0% unsaturated derivative of ovomucoid or soybean trypsin inhibitor (STI). After polymerization was complete, the hydrogels were reduced to small size by pressing through Nylon sieves, washed with distilled water until unreacted compounds were completely removed, and subjected to lyophilic drying. The fraction with a diameter ≤ 0.05mm was taken. Protein/peptide-containing preparations were obtained by swelling in an aqueous solution of protein/peptide with a concentration of 3.0 mg/ml of lyophilically dried unmodified or modified polyacrylamide hydrogel particles.

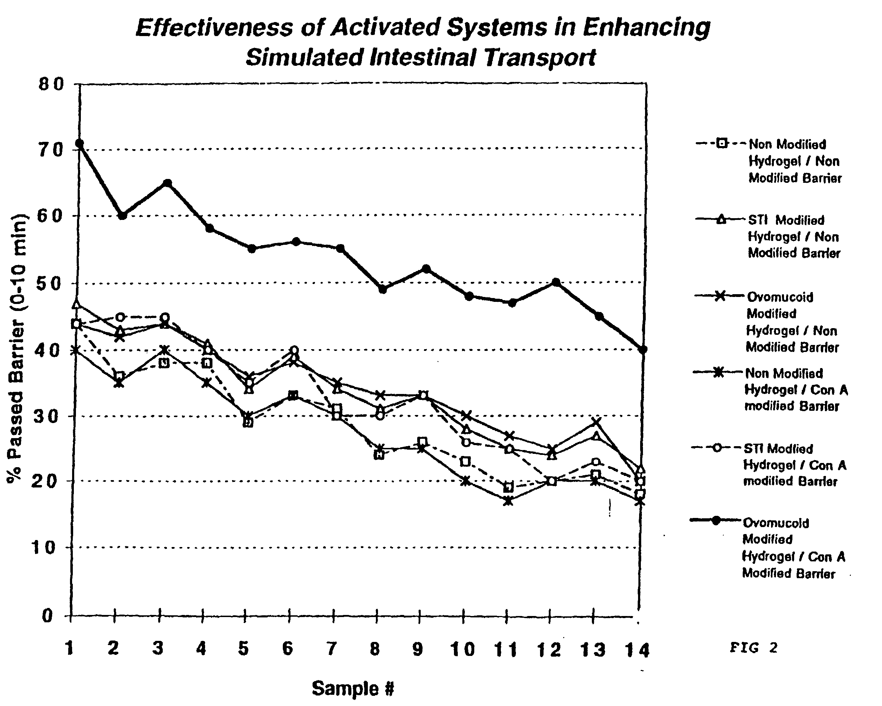

The reactor used to study the permeability of protein/peptides represents a vessel with an inner diameter of 2.0 cm and the walls with a thickness of 0.7 cm made of polyurethane foam, which provided the mechanical strength of the system (Fig. 1). The open pores of the polyurethane reactor were Con-A, which simulated the mucosal membrane of the intestine. The walls of the reactor were impregnated with an aqueous solution of the monomers (3 % of acrylamide, 0.3 % of N,N'-methylenebisacrylamide) and a polymerization catalyst. After polymerization, a viscous polyacrylamide gel not exhibiting mechanical strength was obtained, which was uniformly distributed in.the polyurethane pores. To produce polyacrylamide hydrogel containing Con-A, to simulate the mucosal membrane of the intestine, the initial solution of monomers contained 2 mg of Con-A per ml. of the polymerization solution. Con-A was slightly acylated with acryloylchloride (molar ratio Con-A: acryloylchloride = 1:10).

The studied 2 g of protein/peptide-containing gel was placed inside the reactor, which was placed in a glass vessel containing 12 ml of distilled water. The concentration of the released protein/peptide was determined by measuring the optical density of the solution using a Hitachi-3410 (Japan) spectrophotomete instrument at a wavelength of 280 nm.

The concentration of protein/peptide at equilibrium will be equal to 1/7 of the initial concentration (in units of mg protein/ml of hydrogel) placed into the 2ml reactor. The solution in the glass vessel (permeate side of the barrier) is continuously recirculated through the spectrophotometer which has a hold up volume of about -0.3ml. Any standard laboratory low shear analytical type fluid pump may be employed to carry out the measurement in this fashion.

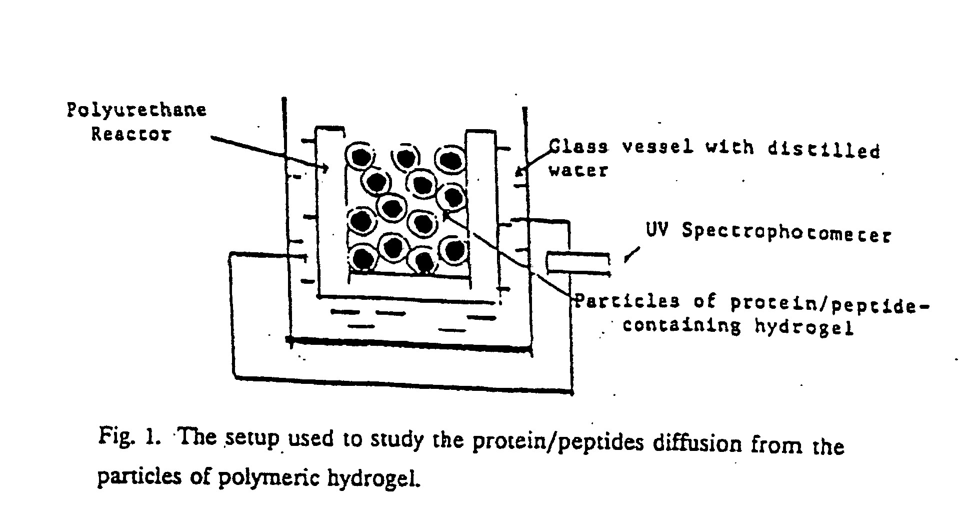

Each value reported in Tables 8-13 is the mean of two different measurements.

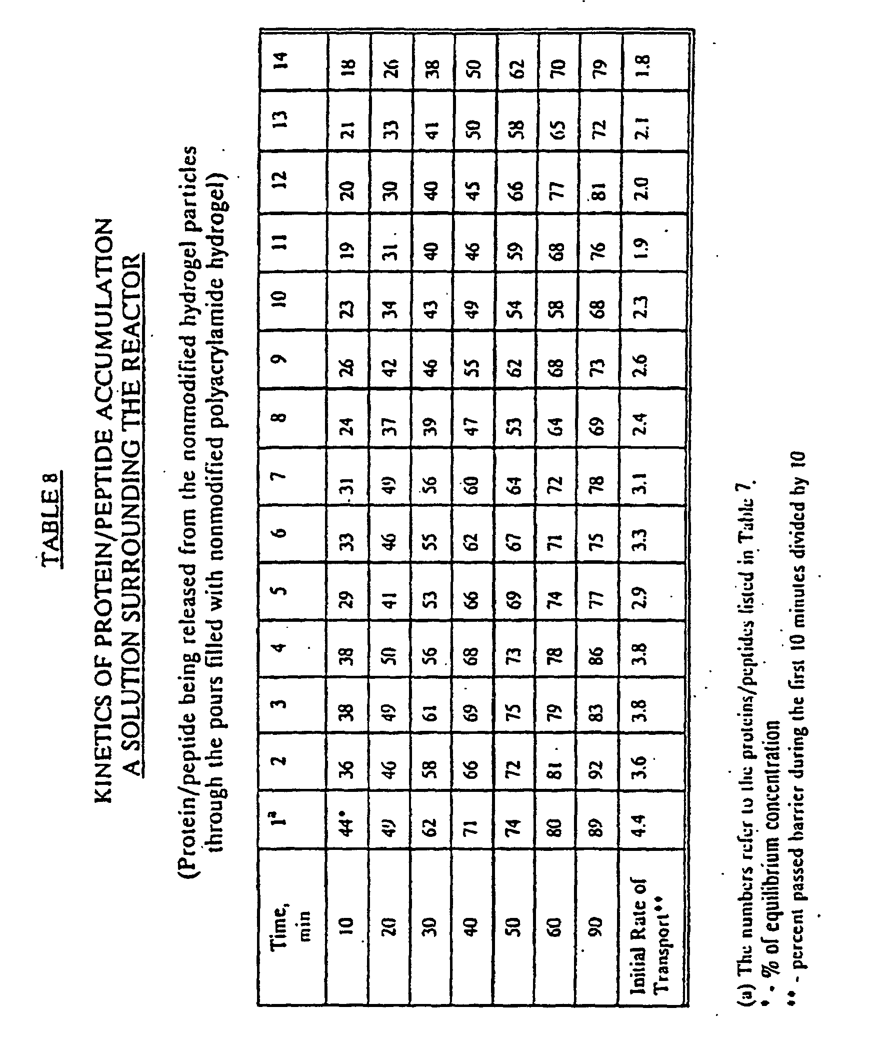

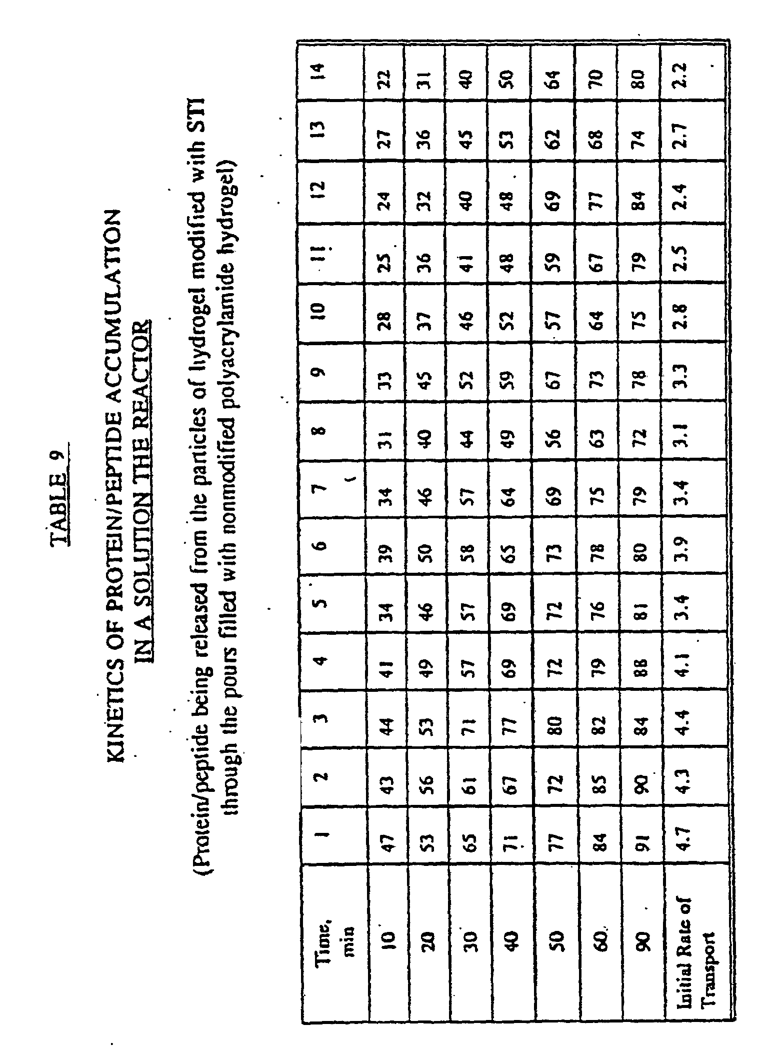

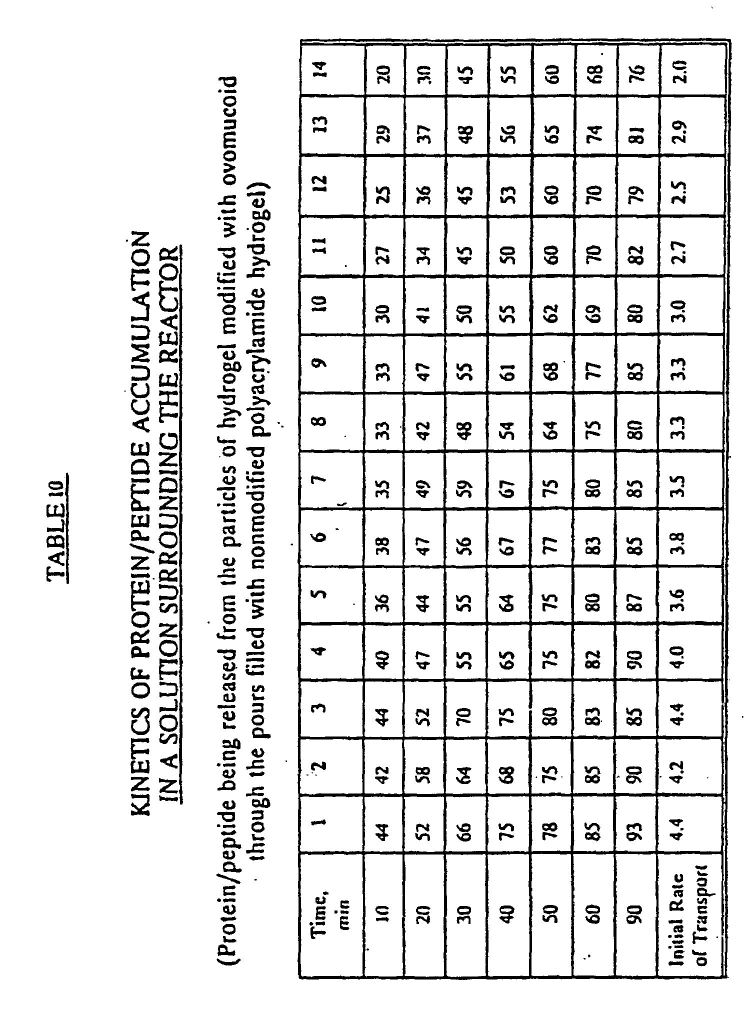

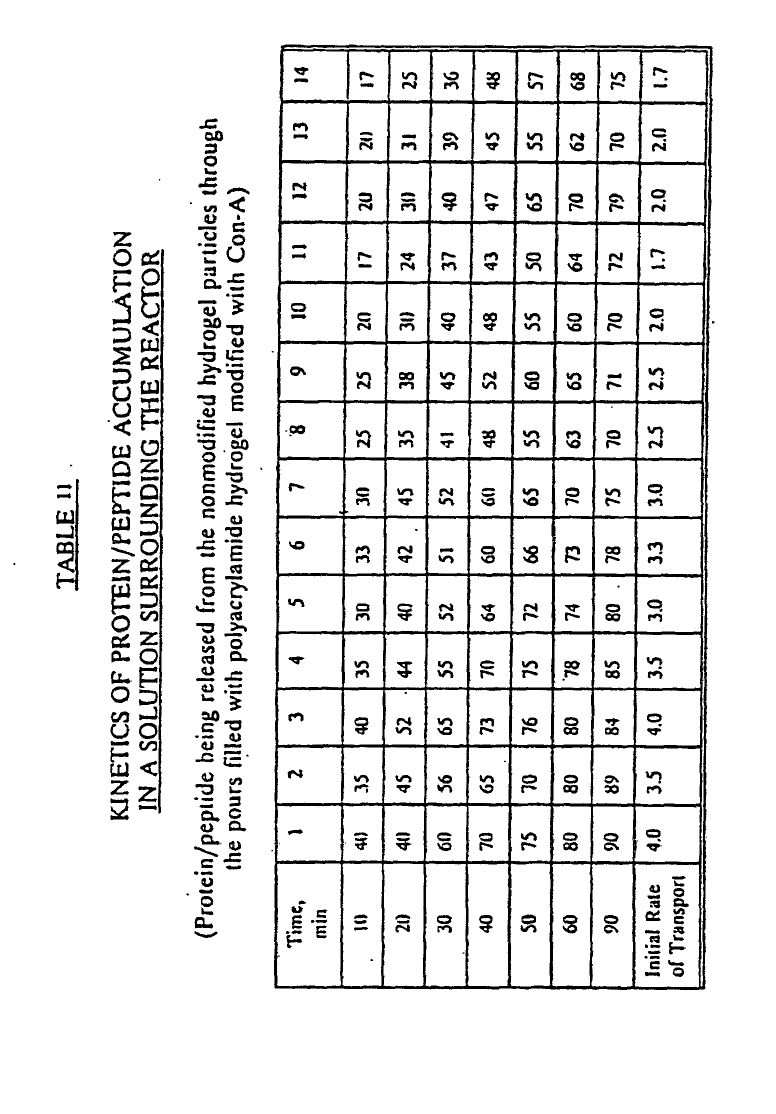

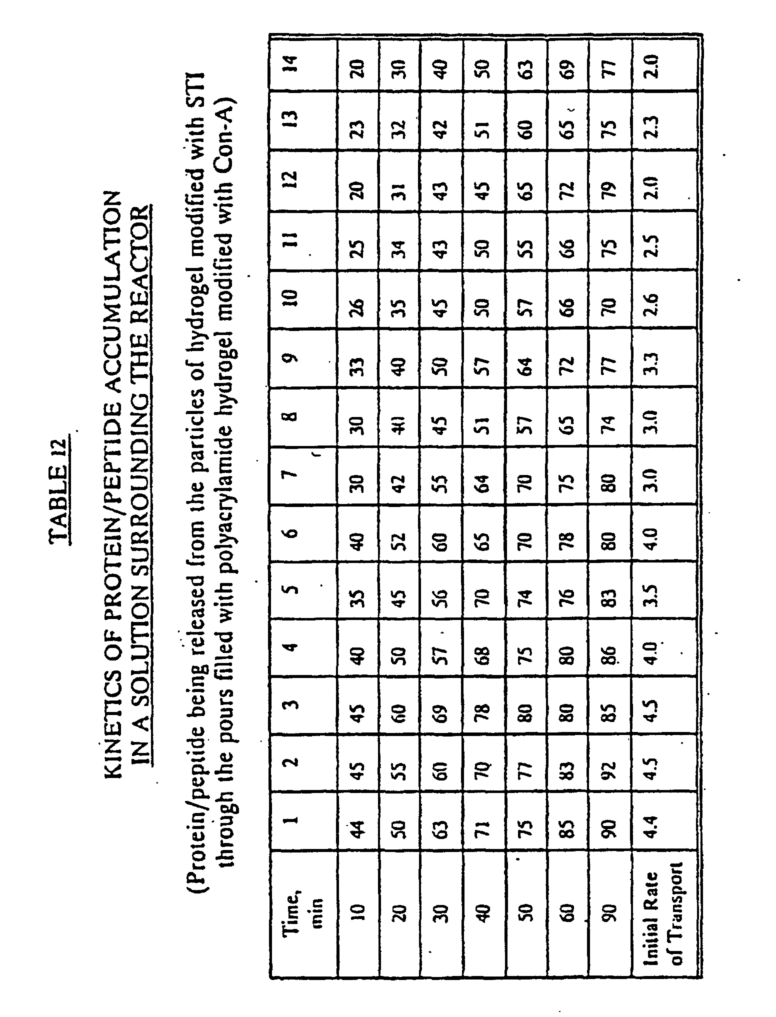

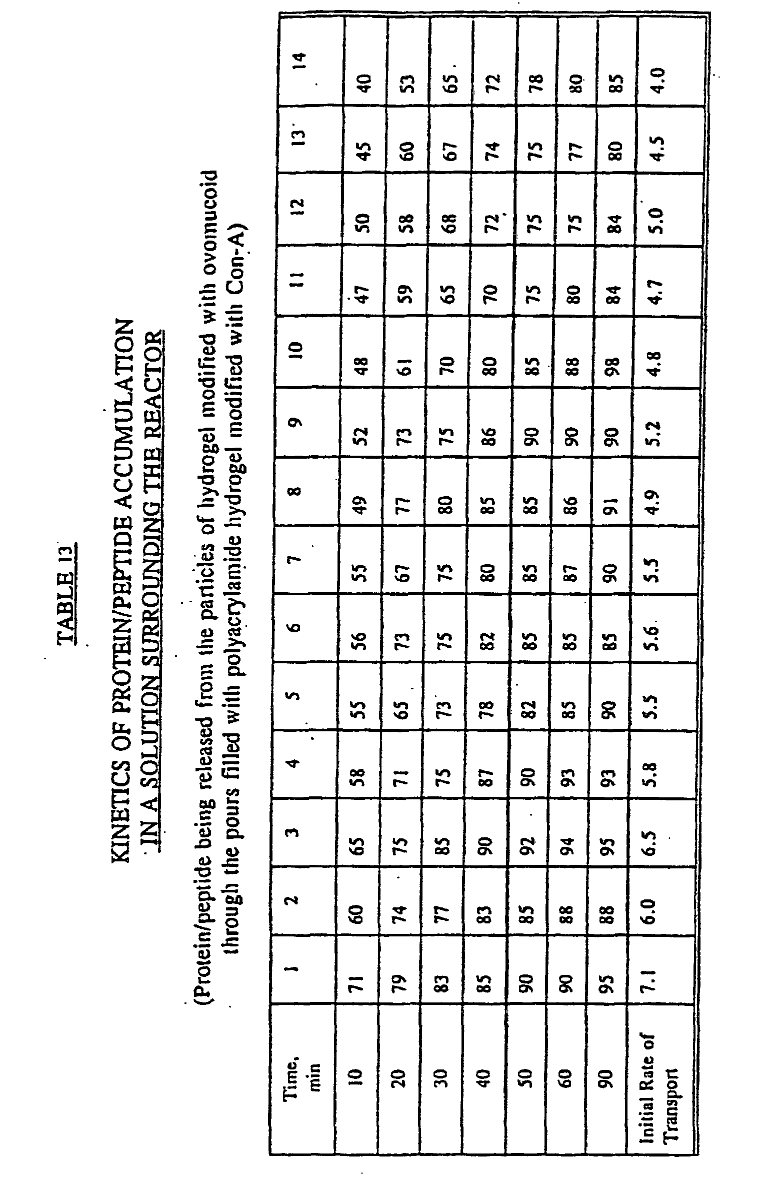

Critical to evaluating the results of the transport studies shown in table 8-14 are the initial diffusion rates (the amount of protein passing through the barrier in the first 10 minutes of the measurement) and effect of the enhanced activity on high molecular weight proteins which are notoriously hard to deliver by any means other than injection. This effect is summarized and illustrated in Figures 2, 3 and 4. In 5 of the 6 combination of delivery gels and barriers studied and reported in tables 8-13, and 14A and represented in the graphs contained in the Figures, at least one key component of the invention required to enhance transport is missing from either the barrier or the delivery system. These are referred to as non-activated systems.

In the sixth combination, ovomucoid is used as the critical binding element in the gel formulation (its role is to both protect the protein and to bind to interactive elements in the intestinal barrier) while Concanavalin A (Con-A) (a well known lectin binding agent) is immobilized within the transport barrier to simulate the lectin binding and other active agents present in various portions of the intestinal barrier. For the purpose of description, this system is referred to as an activated system which, according to the invention, should show an enhancement in transport for proteins contained within the formulation to which the ovomucoid or other binding entity is attached. Table 14B reports a separate study on only two proteins which demonstrates that the activating linkage need not be a part of the inhibitor molecule and may be separately incorporated into the formulation. In this table a palydextran functionality is separately bound onto the hydrogel along with soybean trypsin inhibitor (STI). This system in which this gel is used with the barrier containing Con-A is also an activated system according to the invention.

In Tables 8-14, and Figures 2-4, the sample numbers refer to the same protein as listed in Table 7, though in Table 14B only two proteins are evaluated.

Figure 2 shows the initial transport rates for each system studied for each of the proteins evaluated in order of increasing molecular weight. It is quite evident that for all the non-activated systems (in which the barrier and the delivery gel do not contain bound groups that interact), the amount of protein initially transported is very similar for all the proteins studied. This ranges from about 40-45% in the first 10 minutes of the lowest Mw species to about 17-22% for the highest Mw species. However, when ovomucoid is bound to the delivery gel, and a binding ligand such as Con-A is chemically immobilized in the barrier, the effect on all proteins is dramatic. Initial rates for low Mw species are increased to about 60-70% passage while those for higher Mw species increases to the range of 40-50%.

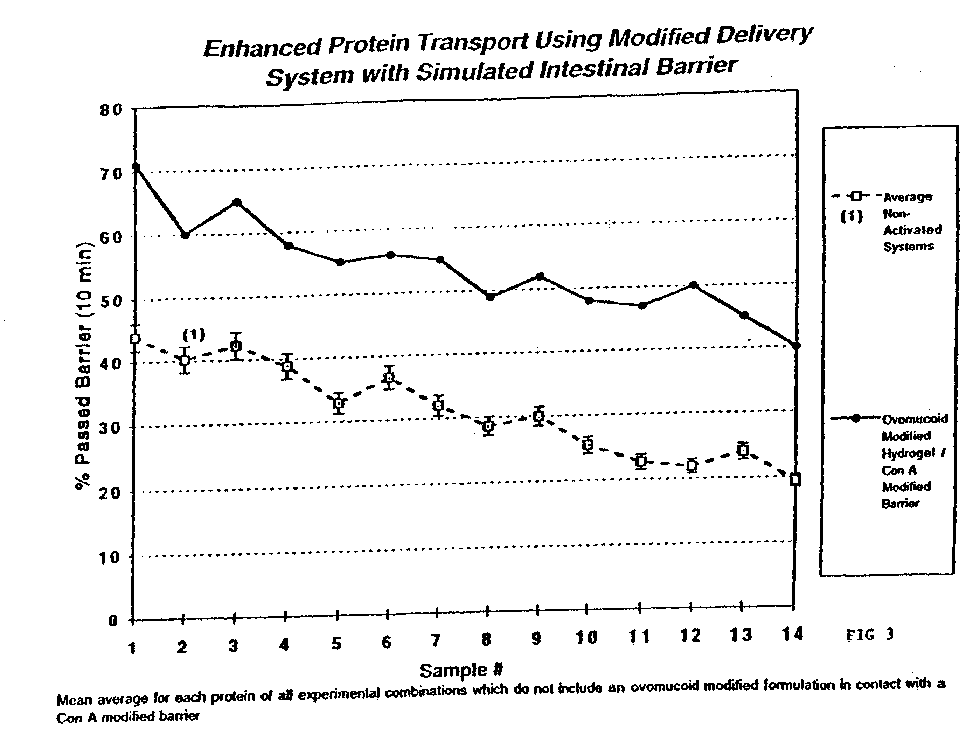

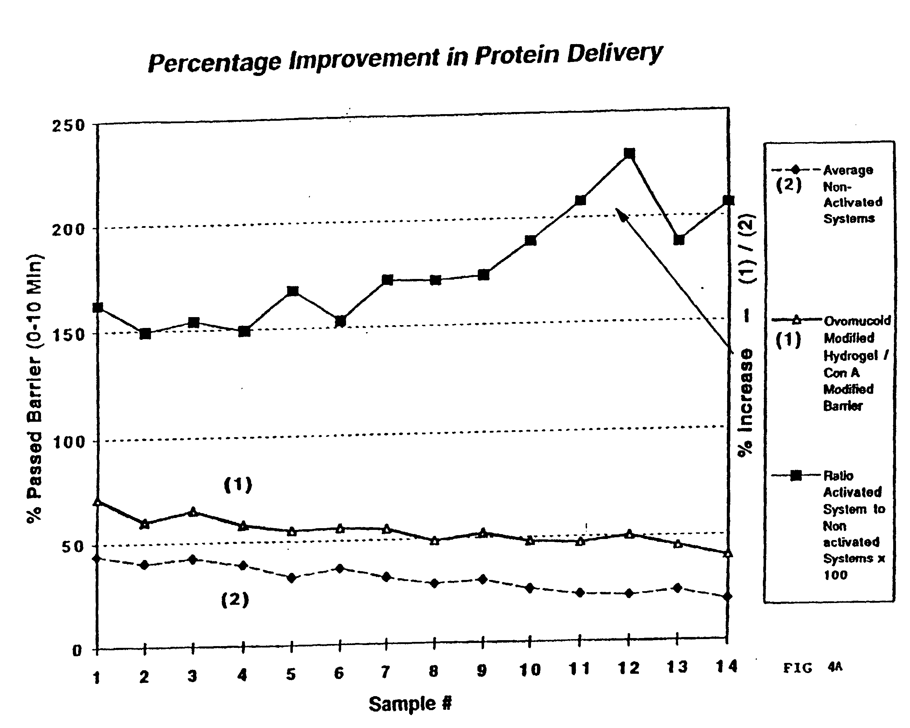

This effect and its magnitude over the range of molecular weights studied is shown clearly in Figures 3 and 4A which show respectively, the effect of the activated system measured against the average of all the non-activated systems (Fig 3) and the same two curves along with the ratio of the activated system to the non-activated systems (Fig 4A). Not only is the effect dramatic for all the proteins studied, but it actually increases at the highest molecular weights so that initial transport of these species is more than 200% that of the same species transported using the non-activated systems.

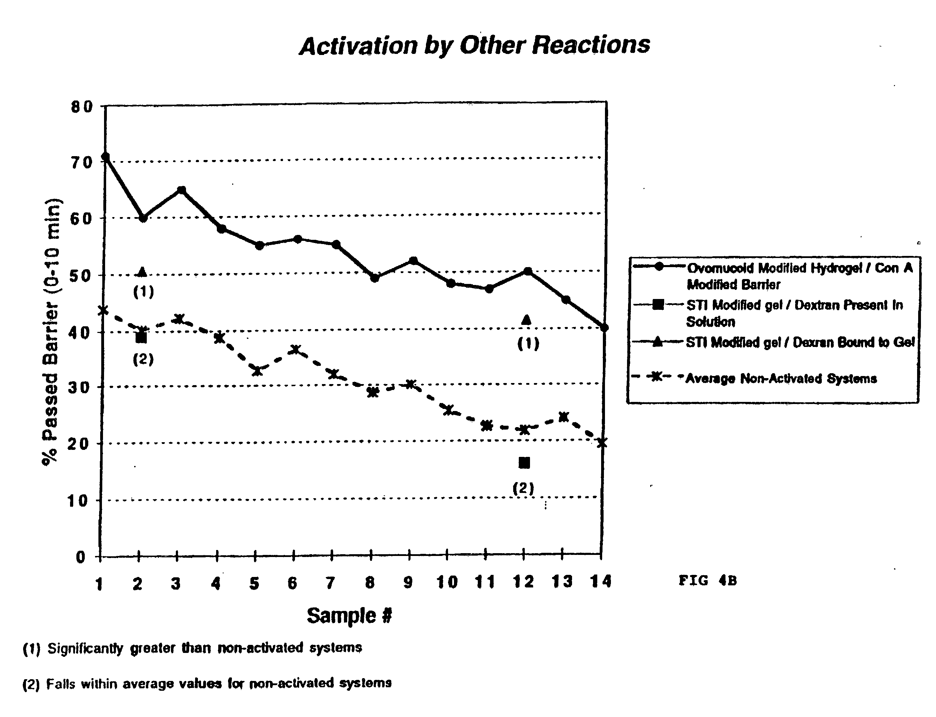

Figure 4B combines the results in Figure 3 with the results illustrated in Table 15. Again only the protein transport in the first 10 minutes were used in the Figure. Samples 2 and 12 were evaluated using two additional combinations of delivery gel and barrier to demonstrate that activation of the gel for enhanced protein delivery may be accomplished by fully following the teachings of the invention and not merely by activating with ovomucoid. In this example, Calcitonin (Sample 2) and Fibrinogen (Sample 12) were evaluated with an STI modified gel delivery system in combination with a Con-A modified barrier (Table 14A). In one case, however, dextran is bound to the STI modified barrier as described. In the other case, it is merely included in non-bound form in the mixture. Clearly, when dextran is bound to the gel, transport of the proteins is enhanced. As in the case of ovomucoid bound to the gel, the dextran/STI bound gel shows more enhancement for the higher molecular weight proteins. This clearly demonstrates the broad scope of the invention, i.e., as long as the hydrogel is modified with an inhibitor and a glucoside, there is an enhancement in the transport of high molecular weight proteins which is both surprising and quite striking. This is represented in Figure 2.

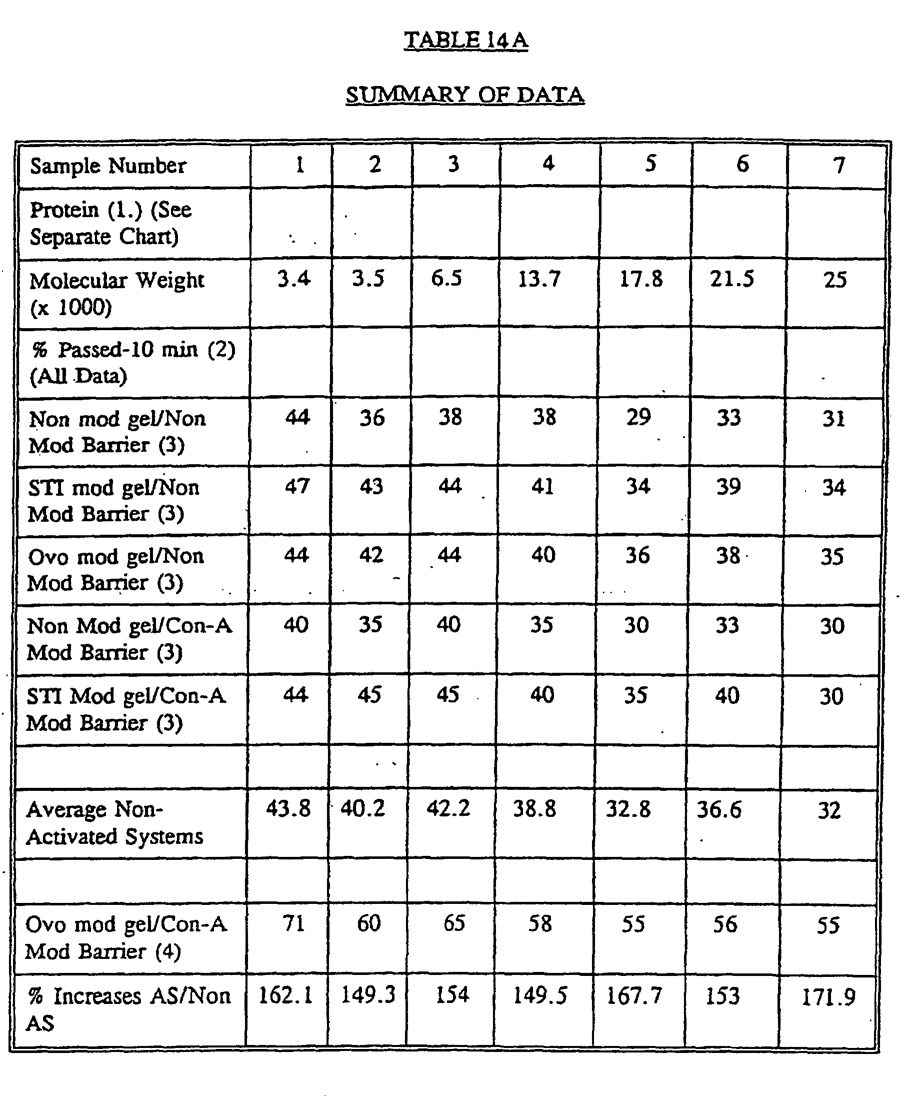

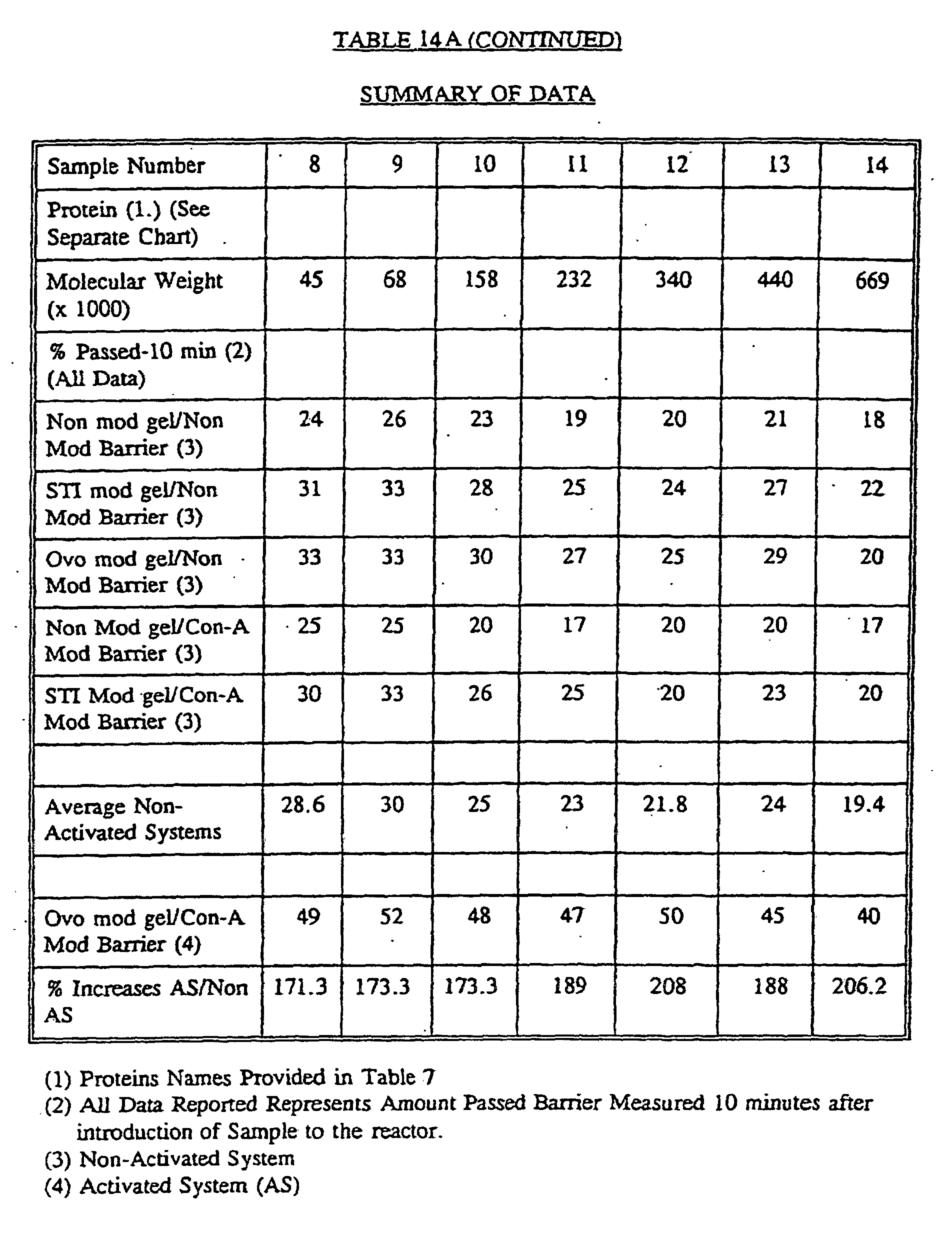

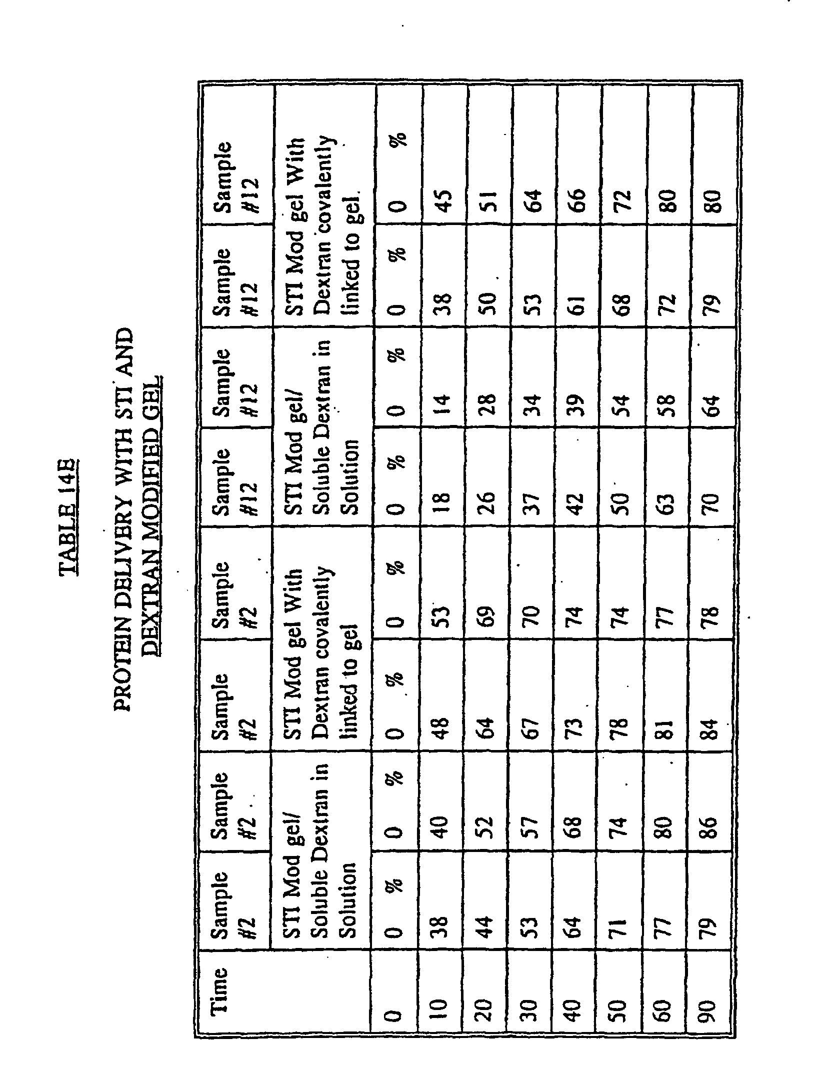

As is seen, that in the case of diffusion through the inert polymeric hydrogel, the rate of protein/peptide accumulation in the solution surrounding the polyurethane vessel is practically independent either of the nature of the inhibitor or the presence of the inhibitor in the polymer particles and is controlled only by the molecular weight of protein/peptide.

Practically similar dependences were observed in the study of protein/peptides release from the polymer particles unmodified and modified by STI and their diffusion through the pores filled with hydrogel with Con-A.

For the polymer particles modified by ovomucoid, the initial rates of protein/peptides accumulation in the surrounding solution accumulation are abnormally high.

Thus, in oral administration, the mechanism of action of protein/peptides immobilized in the particles of a polymer hydrogel modified by ovomucoid involves inhibition of protein/peptides proteolysis due to neutralization of proteolytic enzymes by ovomucoid and increase in the rate of protein/peptides diffusion from the particles of ovomucoid-containing hydrogel through the walls of small intestine. The latter is associated with a specific reaction between the glysoside portion of ovomucoid chemically bound with hydrogel and lectin contained in the mucosal membrane of intestine. This reaction leads to the increase in the local concentration of protein/peptides directly in the musocal membrane and, as a result, the rates of their diffusion to the blood stream grow.

In Table 14B, Dextran was covalently linked to hydrogel employing the procedure described for formulation N2 following Table 5. The apparatus used was the same as represented in Fig. 1. A solution of a protein was poured into the inner polyurethane vessel and measurements were made at the indicated time periods to determine the percentage of the protein that passed from the polyurethane vessel which contained the hydrogel modified as indicated in the Table.

Table 6 shows data of the mmol of Ca²⁺/l of blood in a rabbit after 1 mg of calcitonin was administered subcutaneously and orally in a polyacrylamide gel which had been modified with ovomucoid isolated from the duck egg whites. Procedures described above were used for the preparation of the modified polymers and for absorbing calcitonin into the gel. The concentration of calcium in the blood was determined by a standard procedure using a selective electrode. The data confirms that calcitonin is protected by the ovomucoid-modified hydrogel when it is administered orally.

It is well known, and was noted above, that enzymes, including pepsin, generally decompose all proteins and peptides. Ovomucoid is itself a protein. Therefore, one may expect that ovomucoid may be decomposed when exposed to pepsin. Table 15 shows, however, that ovomucoid, when immobilized in a hydrogel, is not decomposed by pepsin. In the experiments reported in Table 15 10 g of polyacrylamide hydrogels containing 7.1 mg of immobilized ovomucoid per 1 g of hydrogel (prepared as described above) were incubated with 20 ml of pepsin solution (pH 2.0, 0.01 M HCl, 0.2 mg of pepsin/ml) for 0.5; 1.0 and 1.5 hours. After incubation the hydrogels were washed with distilled water and 0.05 M Tris-HCl buffer (pH 7.5) to remove pepsin. Then the hydrogels were incubated with 50 ml of trypsin solution (pH 7.5, 2.3 mg of trypsin/ml) for 1.0 hour and were washed with the same buffer solution (10 x the gel volume). The amount of trypsin bonded to ovomucoid-modified hydrogel was determined as a difference between the initial amount of trypsin and the amount of trypsin in the solution after incubation and washing gels with 10 x the volume of tris buffer. This was compared to gels which were not exposed to pepsin. This data reported in Table 15, shows that the bound ovomucoid (which is a protein) retains most of its trypsin inhibitory activity even after being exposed to the pepsin solution for up to one and one half hours.

**TABLE 15**

| TRYPSIN CAPACITY OF OVOMUCOID-MODIFIED HYDROGEL AFTER EXPOSURE TO PEPSIN | | | | |
|---|---|---|---|---|
| Mg. of Trypsin/10 g of Hydrogel | | | | |
| Run No. | Control (No Pepsin Exposure) | Time of Exposure to Pepsin | | |
| | | 0.5 hr. | 1.0 hr. | 1.5 hr. |
| 1 | 44.1 | 43.5 | 40.8 | 38.7 |
| 2 | 48.3 | 40.8 | 41.7 | 42.3 |
| 3 | 41.2 | 49.1 | 38.7 | 36.1 |
| 4 | 42.1 | 41.9 | 37.4 | 35.4 |
| 5 | 41.7 | 44.5 | 40.2 | 35.6 |
| Mean | 43.5 | 44.0 | 39.8 | 37.6 |

Further illustrative examples of therapeutic or physiologically active materials which have relatively low bioavailability when administered to a human or an animal are listed in Tables 16 and 17 below. These physiologically active materials may be conveniently administered employing the delivery system and thus the method of this invention. The bioavailability of these materials are increased when they are administered using the method of this invention.

**TABLE 16a**

| THERAPEUTICS OF LOW ORAL BIOAVAILABILITY | | | |
|---|---|---|---|
| **THERAPEUTIC** | **Oral Avail. (%)** | **THERAPEUTIC** | **Oral Avail. (%)** |
| Acyclovir | 15 - 30 | Ethosuximide | |
| Alfentanil | | Fentanyl | |
| Alprazolam | | Flurazepam | |
| Alprenolol | 8.6 | Gentamicin | |
| Amikacin | | Gold Sodium Thiomalate | |
| Amphoterican B | | Heparin | |
| Altiacurium | | Hydralazine | 16 |
| Azlocillin | | Kanamycin | |
| Bleomycin | | Labetalol | 20 |
| Busulfan | | Lorcainide (dose dependent) | |
| Carbenicillin | | Methicillin | |
| Carmustine | | Methohexital | |
| Cefamandole | | Methyldopa | 25 |
| Cefazolin | | Mezlocillin | |
| Cefonicid | | Naloxone | 2 |
| Cefoperazone | | Neostigmine | |
| Ceforanide | | Netilmicin | |
| Cefotaxime | | Nicotine | |
| Cefoxitin | | Pancuronium | |
| Ceftazidime | | Pipetacillin | |
| Ceftizoxime | | Prazepam | |
| Cefuroxime | | Pyridostigmine | 14 |
| Cephalothin | | Quinine | |
| Cephapirin | | Rifampin | |
| Chlorambucil | | Streptomycin | |
| Chlorothiazide (dose dependent) | 1 g 9 50 mg 56 | Terbutaline | 15 |
| Cisplatin | | Thiopental | |
| Clorazepate | | Ticarcillin | |
| Cocaine | 0.5 | Tobramycin | |
| Cytarabine | 20 | Tubocurarine | |
| Doxorubicin | | Vancomycin | |
| Edrophonium | | | |

(Goodman & Gillmans The Pharmacological Basis of Thetapeutics*, Hardman, J.G. (ed) & Limbird, L.E. (ed) Ninth Edition (1996) McGraw-Hill, New York)

| **THERAPEUTIC** | **Oral Avail. (%)** | **THERAPEUTIC** | **Oral Avail. (%)** |
|---|---|---|---|
| Aldesteukin | | Lincomycin | 20 - 30 |
| Alteplase (t-PA) | | Lisinopril | 25 |
| Amiloride | | Lovastatin | < 5 |
| Anistreplase | | Mercaptopurine (administered with allopurinol) | 12 60 |
| Auranofin | 15 - 25 | Morphine | 24 |
| Aztreonam | < 1 | Moxalactam | 3 |
| Bretylium | 23 | Nalbuphine | 16 |
| Bromocriptine | 3 - 6 | Naltrexone | 5 - 40 |
| Budesonide Nasel | 12 21 | Nicardipine | 18 |
| Bupivacaine | | Nimodipine | 10 |
| Bupropion | | Nitrendipine | 11 |
| Capreomycin | | Nitroglycerine Sublingual Topical | < 1 38 72 |
| Carboplatin | | Octreotide SubQ Intranasal | <2 100 25 |
| Carvedilol | 25 | Paclitaxel | Low |
| Cefaclor | | Pentamidine | Negligible |
| Ceftriaxone | | Pravastatin | 18 |
| Cinoxacin | | Pyridostigmine | 14 |
| Dobutamine | | Rifabutin | 20 |
| Doxorubicin | 5 | Selegiline | Negligible |
| Esmolol | | Simvastatin | <5 |
| Felodipine | 5 | Spironolactone (after IV Canrecone) | 25 |
| Flumazenil | 20 | Tacrine | 17 |
| Fluorouracil | 28 | Tacrolimus | 16 |
| Ganciclovir | 3 | Terbutaline | 14 |
| Isotretinoin | 25 | Tetrahydrocannabinol Smoking | 4 - 12 2 - 50 |
| Isradipine | 19 | Triamcinolone Acetonide | 23 |
| a) From Goodman and Gilman's The Pharmacological Basis of Therapeutics, 1985. | | | |

**TABLE 16b**

| Further examples of Low Bioavailability Drugs from Cho (U.S. Pat. 4,849,227) not listed specifically in Table 7a or elsewhere in text. | |
|---|---|
| Urokinase | for treating thrombosis |
| Factor VIII | for treating hemophilia |
| Leuprolid | for treating prostrate cancer |
| Gangliocides | for improving neurotransmission |
| Vincrostine | for treating cancer |
| Belomyein | for treating cancer |
| Adrinmycin | for treating cancer |
| Lidocane | for treating cardiac arrhythmia |
| Cephalosporidines | for treating infection |
| Erythromycin | |
| Bretylium Tosylate | |
| Cetiedile | |
| Cyclandelate | |
| Chloramphenicol | |

**TABLE 17**

| SOME PROTEINS AND PEPTIDES OF LOW MOLECULAR WEIGHT AMENABLE TO ORAL DELIVERY BY COMPOSITIONS OF THE INVENTION | | |
|---|---|---|
| | **Name** | **Molecular Weight** |
| 1 | Parathyroid Hormone (PTH) (PTH-RP) | 20194 |
| 2 | Selenoprotein P (Selanoprotein P Precursor) | 42,614 |
| 3 | Cystatin B (Liver Thiol Proteinase Inhibitor) | 11,174 |
| 4 | Tumor Invasion-inhibiting Factor 2 (TIIF-2) | |
| 5 | Tat Protein of HIV-1 | 9758 |
| 6 | HIV Protease | |
| 7 | Transactivating Regulatory Protein (BPC-157) | 40,000 |
| 8 | Megakaryocyte Stimulatory Factor (MGDF) | 37822 |
| 9 | Granulocyte-macrophage Colony Stimulating Factor Precursor (GM-CSF) | 16295 |
| 10 | Soluble Epoxide Hydrolase (CEH) | 62633 |
| 11 | IL-1 Receptor Agonist (EL-1 - RA) | 20,055 |
| 12 | Calcitonin | 15467 |
| 13 | Tumor Necrosis Factor (TNF) (also Catchetin) | 25644 |
| 14 | IFN Gamma (Interferon Gamma Precursor) | 19348 |
| 15 | Interleuian-1 (IL-2) (TCGF) T Cell Growth Factor | 17628 |
| 16* | Interleukin-3 (IL-3) | 17233 |
| 17 | Interleukin-4 (IL-4) (BSF-1) (B Cell Stimulatory Factor) | 17492 |
| 18 | Interleukin-7 (IL-7) | 20,128 |
| 19 | Hemopoietic Cell Growth Factors | |
| 20 | Erythropoietin (EPO-R) Erythropoietin Receptor Precursor | 55065 |
| 21 | Ovomucoids (from Duck, Chicken, Turkey and Other Fowl) | ∼ 28,000 |
| 22** | Meganins (Synthetic and Natural) | ∼ 1000-3000 |

| | | |
|---|---|---|
| *Includes role of IL-3 as a precursor, multipotential colony-stimulating factor, hemopoietic growth factor, P-cell stimulating factor and mast-cell growth factor (MCGF) | | |
| **Meganins are low molecular weight peptides which may be divided from the skin of frogs or synthesized directly. They have shown potential as antibiotics but are only useful topically because of their low oral availability. | | |

## Claims

1. A therapeutic-containing composition adapted for the oral administration of a biologically active material other than insulin comprising
(a) a water insoluble, but water swellable, hydrophilic polymer which is chemically modified to contain at least one proteolytic enzyme inhibitor and a chemical functionality which has an interactive affinity for target receptors located on the transport barrier walls of the digestive track of the intended animal specie, which chemical functionality is a lectin binding agent or a glycoside;
(b) one or more therapeutic materials selected from a growth hormone, an interleukin, a blood cell growth stimulating factor, erithropoitin (EPO), parathyroid hormone (PTH), selenoprotein P, cystatin B, megakaryocyte simulatory factor (MGDF), granulocyte macrophage colony stimulating factor, tumor invasion inhibiting factors, transacting regulatory proteins (TAT's) of HIV and other retroviruses, and transactivating regulator protein BPC 157, fat reducing hormones, calcitonin, glucagon; and
(c) water.

2. A composition of claim 1, wherein said polymer is a partially crosslinked gel forming a hydrophilic polymer.

3. A composition of claim 1, wherein the proteolytic enzyme inhibitor is ovomucoid and it is functionalized by reacting ovomucoid with a polymerizable material.

4. A composition of claim 1, wherein the affinity functionality is a sugar moiety which is bound to the hydrophilic polymer.

5. A composition of claims 1, 2, 3, or 4 wherein the hydrophilic polymer is a homopolymer or a copolymer prepared from one or more monomers selected from acrylic and methacrylic acids, acrylamide, methacrylamide, hydroxyethylacrylate and methacrylate, and vinylpyrrolidones, the therapeutic is a protein or a peptide with a molecular weight of less than 100,000 daltons and the inhibitor is functionalized by reacting it with acryloylchloride.

6. The composition of claim 1 wherein the affinity functionality is part of the inhibitor of proteolytic enzymes and is represented by lectin binding groups of sugars or related saccharides.

7. A composition of claims 1, 3, 4, 5, or 6, which is enclosed within an enteric protective coating and optionally contains pharmacologically acceptable antimicrobial agents, stabilizers, salts and other formulation adjuvants.

8. A method of preparing a composition of claim 1 comprising the steps of
(a) functionalizing a proteolytic enzymes inhibitor by reacting said inhibitor with a compound having a polymerizable vinyl group and a functional group which is reactive with said inhibitor,
(b) polymerizing a mixture comprising 75 to 99 weight percent of one or more polymerizable monomers, 0.1 to 20 weight percent of a crosslinking agent and 0.01 to 5 weight percent of a functionalized proteolytic enzyme inhibitor obtained in step (a) to yield a hydrophilic polymer, and .
(c) immersing the hydrophilic polymer in an aqueous solution of a therapeutic material selected from a growth hormone, an interleukin, a blood cell growth stimulating factor, erithropoitin (EPO), parathyroid hormone (PTH), selenoprotein P, cystatin B, megakaryocyte simulatory factor (MGDF), granulocyte macrophage colony stimulating factor, tumor invasion inhibiting factors, transacting regulatory proteins (TAT's) of HIV and other retroviruses, and transactivating regular protein BPC 157, fat reducing hormones, calcitonin and glucagons.

9. A composition of claim 1, wherein the polymer is a crosslinked hydrophilic gel and the inhibitor is physically entrapped within the polymer matrix.

## Patentansprüche

1. Therapeutikumhaltige Zusammensetzung, die an die orale Verabreichung eines anderen biologisch aktiven Materials als Insulin angepaßt ist, umfassend
(a) ein wasserunlösliches, aber wasserquellbares, hydrophiles Polymer, daß chemisch modifiziert ist, um mindestens einen Inhibitor eines proteolytischen Enzyms und eine chemische Funktionalität zu enthalten, die eine interaktive Affinität auf Target-Rezeptoren aufweist, die sich auf den Transportsperrwänden des Verdauungstrakts der bestimmungsgemäßen Tierart befinden, wobei die chemische Funktionalität ein lectinbindendes Mittel oder ein Glykosid ist;
(b) ein oder mehr therapeutische Materialien, die aus einem Wachstumshormon, einem Interleukin, einem das Blutzellenwachstum stimulierenden Faktor, Erythropoietin (EPO), parathyroidem Hormon (PTH), Selenoprotein P, Cystatin B, Megakaryozytenstimulierungsfaktor (MGDF), Granulozyten-Makrophagen-koloniestimulierendem Faktor, Tumorinvasionshemmfaktoren, transaktivierenden Regulatorproteinen (TAT) des HIV und anderer Retroviren und dem transaktivierenden Regulatorprotein BPC 157, fettreduzierenden Hormonen, Calcitonin und Glucagon ausgewählt sind, und
(c) Wasser.

2. Zusammensetzung des Anspruchs 1, wobei das Polymer ein teilweise vernetztes Gel ist, das ein hydrophiles Polymer bildet.

3. Zusammensetzung des Anspruchs 1, wobei der Inhibitor des proteolytischen Enzyms Ovomucoid ist und er durch Umsetzen von Ovomucoid mit einem polymerisierbaren Material funktionalisiert wurde.

4. Zusammensetzung des Anspruchs 1, wobei die Affinitätsfunktionalität eine Zuckerstruktureinheit ist, die an das hydrophile Polymer gebunden ist.

5. Zusammensetzung der Ansprüche 1, 2, 3 oder 4, wobei das hydrophile Polymer ein Homopolymer oder ein Copolymer ist, das aus einem oder mehreren aus Acryl- und Methacrylsäure, Acrylamid, Methacrylamid, Hydroxyethylacrylat und -methacrylat und Vinylpyrrolidonen ausgewählten Monomeren hergestellt wurde, das Therapeutikum ein Protein oder ein Peptid mit einem Molekulargewicht von weniger als 100 000 Dalton ist und der Inhibitor durch sein Umsetzen mit Acryloylchlorid funktionalisiert wurde.

6. Zusammensetzung des Anspruchs 1, wobei die Affinitätsfunktionalität Teil des Inhibitors proteolytischer Enzyme ist und durch lectinbindende Gruppen von Zuckern oder verwandten Sacchariden dargestellt wird.

7. Zusammensetzung der Ansprüche 1, 3, 4, 5 oder 6, die mit einer magensaftresistenten Beschichtung umschlossen ist und gegebenenfalls pharmakologisch annehmbare antimikrobielle Mittel, Stabilisatoren, Salze und andere Formulierungshilfsmittel enthält.

8. Verfahren zum Herstellen einer Zusammensetzung des Anspruchs 1, umfassend die Schritte des
(a) Funktionalisierens eines Inhibitors proteolytischer Enzyme durch Umsetzen des Inhibitors mit einer Verbindung, die eine polymerisierbare Vinylgruppe und eine funktionelle Gruppe aufweist, die gegenüber dem Inhibitor reaktionsfähig ist,
(b) Polymerisierens eines Gemisches, das 75 bis 99 Gewichtsprozent eines oder mehrerer polymerisierbarer Monomeren, 0,1 bis 20 Gewichtsprozent eines Vernetzungsmittels und 0,01 bis 5 Gewichtsprozent eines in Schritt (a) erhaltenen funktionalisierten Inhibitors eines proteolytischen Enzyms umfaßt, unter Liefern eines hydrophilen Polymers und
(c) Tauchens des hydrophilen Polymers in eine wäßrige Lösung eines therapeutischen Materials, das aus einem Wachstumshormon, einem Interleukin, einem das Blutzellenwachstum stimulierenden Faktor, Erythropoietin (EPO), parathyroidem Hormon (PTH), Selenoprotein P, Cystatin B, Megakaryozytenstimulierungsfaktor (MGDF), Granulozyten-Makrophagen-koloniestimulierendem Faktor, Tumorinvasionshemmfaktoren, transaktivierenden Regulatorproteinen (TAT) des HIV und anderer Retroviren und dem transaktivierenden Regulatorprotein BPC 157, fettreduzierenden Hormonen, Calcitonin und Glucagon ausgewählt ist.

9. Zusammensetzung des Anspruchs 1, wobei das Polymer ein vernetztes hydrophiles Gel ist und der Inhibitor innerhalb der Polymermatrix physikalisch eingeschlossen ist.

## Revendications

1. Composition contenant un agent thérapeutique, adaptée à l'administration orale d'un matériau biologiquement actif autre que l'insuline, et comprenant :
(a) un polymère hydrophile insoluble dans l'eau mais pouvant gonfler dans l'eau, qui est chimiquement modifié de façon à contenir au moins un inhibiteur d'enzyme protéolytique et une fonctionnalité chimique qui a une affinité interactive pour des récepteurs cibles situés sur les parois à effet de barrière de transport du tube digestif de l'espèce animale prévue, laquelle fonctionnalité chimique est un agent de liaison de la lectine ou un glycoside ;
(b) un ou plusieurs matériaux thérapeutiques choisis parmi une hormone de croissance, une interleukine, un facteur stimulant la croissance des globules sanguins, l'érythropoïétine (EPO), l'hormone parathyroïdienne (PTH), la sélénoprotéine P, la cystatine B, le facteur stimulant les mégacaryocytes (MGDF), le facteur stimulant la formation de colonies de macrophages ou de granulocytes, les facteurs inhibant l'invasion tumorale, les protéines régulatrices de transaction (TAT) du VIH et d'autres rétrovirus, et la protéine régulatrice de transactivation BPC 157, les hormones réductrices de graisses, la calcitonine, le glucagon ; et
(c) de l'eau.

2. Composition selon la revendication 1, dans laquelle ledit polymère est un gel partiellement réticulé, formant un polymère hydrophile.

3. Composition selon la revendication 1, dans laquelle l'inhibiteur d'enzymes protéolytiques est l'ovomucoïde et est fonctionnalisé par réaction de l'ovomucoïde avec un matériau polymérisable.

4. Composition selon la revendication 1, dans laquelle la fonctionnalité d'affinité est un fragment sucre qui est lié au polymère hydrophile.

5. Composition selon les revendications 1, 2, 3 ou 4, dans laquelle le polymère hydrophile est un homopolymère ou un copolymère préparé à partir d'un ou plusieurs monomères choisis parmi l'acide acrylique et l'acide méthacrylique, l'acrylamide, le méthacrylamide, l'acrylate et le méthacrylate d'hydroxyéthyle, et les vinylpyrrolidones, l'agent thérapeutique est une protéine ou un peptide ayant une masse moléculaire inférieure à 100 000 daltons, et l'inhibiteur est fonctionnalisé par réaction avec du chlorure d'acryloyle.

6. Composition selon la revendication 1, dans laquelle la fonctionnalité d'affinité fait partie de l'inhibiteur des enzymes protéolytiques et est représentée par des groupes de liaison à la lectine de sucres ou de saccharides apparentés.

7. Composition selon les revendications 1, 3, 4, 5 ou 6, qui est enfermée dans un enrobage gastrorésistant, et éventuellement contient des agents antimicrobiens, stabilisants, sels ou autres adjuvants de formulation, acceptables d'un point de vue pharmaceutique.

8. Procédé de préparation d'une composition selon la revendication 1, qui comprend les étapes de :
(a) fonctionnalisation d'un inhibiteur d'enzymes protéolytiques par réaction dudit inhibiteur avec un composé ayant un groupe vinyle polymérisable et un groupe fonctionnel qui est réactif avec ledit inhibiteur,
(b) polymérisation d'un mélange comprenant 75 à 99 % en poids d'un ou plusieurs monomères polymérisables, 0,1 à 20 % en poids d'un agent de réticulation et 0,01 à 5 % en poids d'un inhibiteur d'enzymes protéolytiques fonctionnalisé obtenu dans l'étape (a), pour donner un polymère hydrophile, et
(c) immersion du polymère hydrophile dans une solution aqueuse d'un matériau thérapeutique choisi parmi une hormone de croissance, une interleukine, un facteur stimulant la croissance des globules sanguins, l'érythropoïétine (EPO), l'hormone parathyroïdienne (PTH), la sélénoprotéine P, la cystatine B, le facteur stimulant les mégacaryocytes (MGDF), le facteur stimulant la formation de colonies de macrophages ou de granulocytes, les facteurs inhibant l'invasion des tumeurs, les protéines régulatrices de transaction (TAT) du VIH et d'autres rétrovirus, et la protéine régulatrice de transactivation BPC 157, les hormones réductrices de graisses, la calcitonine et les glucagons.

9. Composition selon la revendication 1, dans laquelle le polymère est un gel hydrophile réticulé, et l'inhibiteur est physiquement enfermé à l'intérieur de la matrice polymère.
